(19) Europäisches Patentamt
European Patent Office
Office européen des brevets

(11) Numéro de publication: **0 239 476 B1**

(12) **FASCICULE DE BREVET EUROPEEN**

(45) Date de publication du fascicule du brevet: **24.10.90**

(21) Numéro de dépôt: **87400593.7**

(22) Date de dépôt: **17.03.87**

(51) Int. Cl.[5]: **C 07 D 471/04,** A 61 K 31/435 // (C07D471/04, 221:00, 209:00)

# (54) Dérivés du pyridoindole, leur application à titre de médicaments et les compositions les renfermant.

(30) Priorité: **17.03.86 FR 8604202**

(43) Date de publication de la demande: **30.09.87 Bulletin 87/40**

(45) Mention de la délivrance du brevet: **24.10.90 Bulletin 90/43**

(84) Etats contractants désignés: **AT BE CH DE ES FR GB GR IT LI LU NL SE**

(56) Documents cités:
**EP-A-0 197 829**
**FR-A-2 327 783**
**FR-A-2 422 662**

(73) Titulaire: **SANOFI**
**40, Avenue George V**
**F-75008 Paris (FR)**

(73) Titulaire: **CENTRE NATIONAL DE LA RECHERCHE SCIENTIFIQUE (CNRS)**
**15, Quai Anatole France**
**F-75700 Paris Cedex 07 (FR)**

(72) Inventeur: **Bisagni, Emile**
**16, rue Bossuet**
**F-91400 - Orsay (FR)**
Inventeur: **Chi Hung, Nguyen**
**7, allée des Amonts**
**F-91940 - Les Ulis (FR)**
Inventeur: **Pepin, Odile**
**Barthou, Montjoire**
**F-31380 Montastruc la Conseillère (FR)**

(74) Mandataire: **Polus, Camille et al**
**c/o Cabinet Lavoix 2, Place d'Estienne d'Orves**
**F-75441 Paris Cedex 09 (FR)**

**EP 0 239 476 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

## Description

L'invention est relative à de nouveaux dérivés du 5-H Pyrido [4,3-b] indole, leur procédé de préparation, leur application à titre de médicament et les compositions les renfermant.

Ces composés, qui présentent des propriétés antitumorales intéressantes, répondent à la formule (I) suivante:

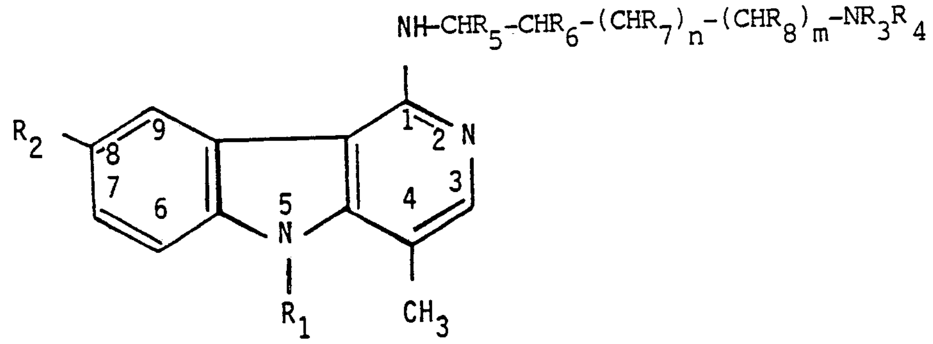

(I)

dans laquelle n et m sont indépendamment 0 ou 1, $R_1$ représente l'hydrogène, ou un groupe alkyle en $C_1$—$C_4$, $R_2$ représente l'hydrogène, un groupe hydroxy ou un groupe alkoxy en $C_1$—$C_4$, $R_3$ et $R_4$ sont chacun indépendamment l'un de l'autre l'hydrogène, un groupe alkyle ou hydroxyalkyle en $C_1$—$C_4$, $R_5$, $R_6$, $R_7$ et $R_8$ représentent indépendamment l'hydrogène, un groupe hydroxy ou un groupe alkyle en $C_1$—$C_4$.

L'invention concerne aussi les formes tautomères de la formule (I) lorsqu'elles existent ainsi que les sels d'addition avec les acides minéraux ou organiques pharmaceutiquement acceptables.

Quelques dérivés de l'amino- 5-H Pyrido [4,3-b] indole ont été antérieurement décrits dans la littérature [C. DUCROCQ, A. CIVIER, J. ANDRELOUISFERT et E. BISAGNI — J. Heterocycl. Chem., *12*, (5), 963—967, (1975), Ch. S. LEE, T. OHTA, K. SHUDO et T. OKAMATO — Heterocycles, *16*, (7), 1081—1084, (1981)].

Mais, ces dérivés ne comportant ni de groupe méthyle en position -4 ni de chaîne alkylaminoalkylaminée en position -1, caractéristiques des composés de formule (I).

En outre, aucune activité thérapeutique n'est signalée pour tous ces dérivés.

L'invention concerne aussi un procédé de préparation des composés de formule (I) caractérisé en ce que:

une méthyl -4 2-H 5-H pyrido [4,3-b] indolone-1 de formule IV

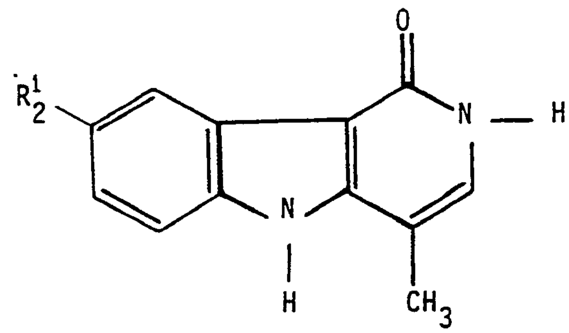

(IV)

dans laquelle R'$_2$ représente l'hydrogène ou un groupe alkoxy en $C_1$—$C_4$, est transformée par action d'un agent chlorant en un chloro-1 méthyl-4 5-H pyrido [4,3-b] indole de formule (V)

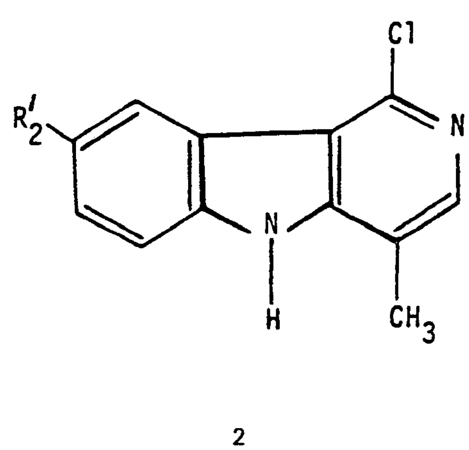

(V)

dans laquelle R'$_2$ représente l'hydrogène ou un groupe alkoxy en $C_1$—$C_4$, puis, éventuellement après alkylation en position 5 au moyen d'un halogénure d'alkyle, pour former le composé de formule VI

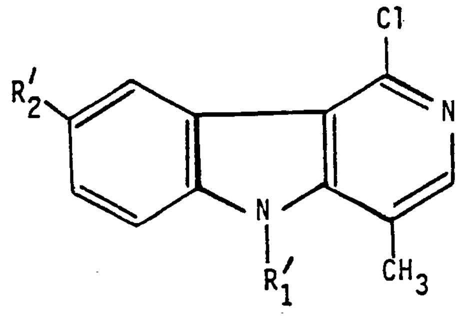

(VI)

dans laquelle R'$_2$ a les mêmes significations que dans la formule (V) et R'$_1$ représente un groupe alkyle en $C_1$—$C_4$, et éventuellement après action d'un acide minéral, tel que HBr ou HI, le composé de formule (V) dans laquelle R'$_2$ représente un groupe alkoxy ou le composé de formule (VI) dans laquelle R'$_2$ représent un groupe alkoxy peut être transformé en chloro-1 hydroxy-8 méthyl-4 5-H pyrido [4,3-b] indole (VI: R'$_1$ = hydrogène ou alkyle en $C_1$—$C_4$; R'$_2$ = un groupe hydroxy),

le composé de formule (V), ou de formule (VI) est condensé avec une amine de formule:

$$H_2N—CHR_5—CHR_6—(CHR_7)_n—(CHR_8)_m—NR_3—R_4$$

dans laquelle n, m, $R_3$, $R_4$, $R_5$, $R_6$, $R_7$ et $R_8$ sont tels que définis pour la formule I.

Le composé de formule IV peut être préparé par exemple de la manière suivante:

L'hydroxy-4 méthyl-5 1-H pyridone-2 ci-dessous dont la synthèse

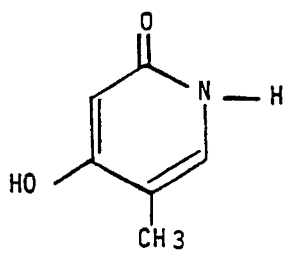

a été décrite par E. BISAGNI et N. CHI HUNG (Synthesis, *1984*, 765), mise à réagir à haute température avec une phénylhydrazine de formule:

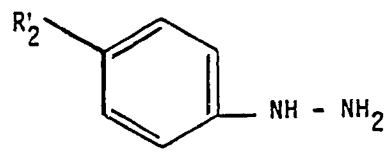

dans laquelle R'$_2$ représente l'hydrogène ou un groupe alkoxy, conduit à la méthyl-4 2-H 5-H pyrido [4,3-b] indolone-1 de formule (IV).

Les exemples non limitatifs suivants sont donnés à titre d'illustration de l'invention.

Exemple 1

Préparation du chloro-1 méthyl-4 5-H pyrido [4,3-b] indole (composé de formule (V) dans lequel R'$_2$ = H).

A) Méthyl-4 2-H 5-H pyrido [4,3-b] indolone-1 (composé de formule IV dans lequel R'$_2$ = H).

Le mélange formé par l'hydroxy-4 méthyl-5 1H-pyridone-2 (4,1 g), la phénylhydrazine fraîchement rectifiée (9,5 ml) et le diphényléther (70 ml) est chauffé à reflux pendant 2 h, en élimitant l'eau formée par un appareil de DEAN-STARK puis en distillant l'excès de phénylhydrazine, et refroidi à la température ambiante. Après addition de 100 ml de toluène, le précipité formé est essoré, lavé au toluène et repris dans 100 ml de dioxanne bouillant, dans lequel il est très peu soluble.

On recueille 5,5 g de microcristaux beige clair.

Rendement: 88%; F> 260°C.

| | | | |
|---|---|---|---|
| Calc. pour $C_{12}H_{10}N_2O$; 0,75 $H_2O$: | C, 68,08; | H, 5,43; | N, 13,24; |
| Trouvé: | C, 68,49; | H, 5,28; | N, 13,31. |

RMN H1 [$(CD_3)_2SO$], δ: 2,32 (s, 3H, $CH_3$), 7,18 (s élargi, 1H, H-3), 7,19—7,52 (m, 3H, H-6 + H-7 + H-8), 8.08 (q, 1H, H-9), 10,94 (s large, 1H, NH-5), 11,75 (s, 1H, NH-2).

B) Chloro-1 méthyl-4 5-H pyrido [4,3-b] indole (composé de formule V dans lequel $R'_2$ = H).

Le mélange de méthyl-4 2-H 5-H pyrido [4,3-b] indolone-1 (1,6 g) dans le dichlorure de l'acide phénylphosphonique (120 ml) est chauffé à 160—165°, sous agitation, pendant 4 h et l'excès d'agent de chloration est évaporé sous pression réduite (2 mm). Le résidu est repris dans 400 ml d'acide chlorhydrique 0,5 N chaud, filtré et le filtrat est alcalinisé par l'ammoniaque. Le précipité obtenu, essoré et séché, est recristallisé dans le xylène pour donner des paillettes incolores (1,3 g).

Rendement: 81%; F> 260°C.

| | | | | |
|---|---|---|---|---|
| Calc. pour $C_{12}H_9ClN_2$: | C, 66,52; | H, 4,19; | N, 12,93; | Cl, 16,36; |
| Trouvé: | C, 66,26; | H, 4,18; | N, 12,91; | Cl, 16,28. |

RMN H1 [$(CD_3)_2SO$]; δ: 2,55 (s, 3H, CH3-4), 7,3—7,73 (m, 3H, H-6 + H-7 + H-8), 8.09 (s large, 1H, H-3), 8,40 (d, 1H, H-9), 12,11 (s large, 1H, NH-5).

## Exemple 2

Préparation du chloro-1 méthoxy-8 methyl-4 5-H pyrido [4,3-b] indole (composé de formule (V) dans lequel $R'_2$ = —$OCH_3$).

A) Méthyl-4 méthoxy-8 2-H 5-H pyrido [4,3-b] indolone-1 (composé de formule (IV) dans lequel $R'_2$ = —$OCH_3$).

La réaction est réalisée à partir de la suspension de 2,11 g (16,8 mmoles) d'hydroxy-4 méthyl-5 1-H pyridone-2 dans 60 ml de diphényléther dégazée à l'argon à l'ébullition et de la solution de méthoxy-4 phénylhydrazine (7 g, 50,4 mmoles), séchée sous vide après avoir été libérée de son chlorhydrate dans 80 ml de diphényléther, également dégazée à l'argon, mais à la température ambiante, solution qui est additionnée à la suspension précédente bouillante, maintenue sous argon et sous agitation. Le mélange est chauffé au reflux pendant 6 h, en éliminant l'eau formée par un appareil de DEAN-STARK, refroidi et additionné de 150 ml de Toluène. Le précipité formé est filtré, lavé au Toluène et au pentane, séché et mis en suspension dans 100 ml d'une solution d'hydroxyde de sodium N. Le produit insoluble est filtré puis recristallisé dans le minimum d'éthanol pour donner 660 mg de microcristaux incolores, correspondant au composé attendu partiellement hydraté.

Rendement: 17,1%; F> 260°C.

| | | | |
|---|---|---|---|
| Calc. pour $C_{13}H_{12}N_2O_2$; 0,33 $H_2O$: | C, 66,68; | H, 5,41; | N, 11,97; |
| Trouvé: | C, 66,63; | H, 5,26; | N, 11,99. |

RMN H1 [$(CD_3)_2SO$]; δ: 2,26 (s, 3H, $CH_3$-4), 3,84 (s, 3H, $OCH_3$), 6,94 (q, 1H, H7, $J_{7-6}$ = 8,6Hz, J7-9 = 2,8Hz), 7,07 (s élargi, 1H, H-3), 7,43 (d, 1H, H-6), 7,65 (d, 1H, H-9), 10,82 (s large, 1H, NH-5), 11,55 (s large, 1H, NH-2).

B) Chloro-1 méthyl-4 méthoxy-8 5-H pyrido [4,3-b] indole (composé de formule V dans lequel $R'_2$ = $OCH_3$).

720 mg du composé précédemment obtenu sont chauffés dans l'oxychlorure de phosphore (60 ml) à reflux pendant 3 jours et l'excès d'oxychlorure est évaporé sous pression réduite. Le résidu est repris dans 100 ml d'eau bouillante, le mélange est chauffé à l'ébullition pendant 3 mn et filtré. Le filtrat est alcalinisé à froid par l'ammoniaque et le précipité formé est essoré, séché puis recristallisé dans l'acétonitrile pour donner 580 mg de microcristaux jaunes.

Rendement: 76,5%; F = 243—245°C.

| | | | | |
|---|---|---|---|---|
| Calc. pour $C_{13}H_{11}ClN_2O$: | C, 63,29; | H, 4,49; | N, 11,35; | Cl, 14,37; |
| Trouvé: | C, 63,48; | H, 4,56; | N, 11,15; | Cl, 14,64. |

RMN H1 [$(CD_3)_2SO$]; δ: 2,52 (d, 3H, $CH_3$-4, $J_{CH3-H-3}$ = 1Hz), 3,91 (s, 3H, $OCH_3$), 7,23 (q, 1H, H-7, $J_{7-6}$ = 9Hz, $J_{7-9}$ = 2,5Hz), 7,60 (d, 1H, H-6), 7,87 (d, 1H, H-9), 8,04 (d, 1H, H-3).

## Exemple 3

Préparation du chloro-1 diméthyl-4,5 méthoxy-8 5-H pyrido [4,3-b] indole (composé de formule (VI) dans lequel $R'_1$ = —$CH_3$ et $R'_2$ = —$OCH_3$).

600 mg du chloro-1 méthyl-4 méthoxy-8 5-H pyrido [4,3-b] indole dissous dans 20 ml de N,N-diméthylformamide sont traités par l'iodure de méthyle (0,3 ml; 2 équivalents) en présence de carbonate de potassium (1,7 g; 5 équivalents) à température ambiante pendant cinq heures. Après évaporation du solvant sous pression réduite, le produit obtenu est repris par 20 ml d'eau qui est ensuite acidifiée par une solution aqueuse d'acide chlorhydrique N puis neutralisée par une solution d'ammoniaque. Le précipité obtenu est filtré, lavé à l'eau, séché et recristallisé dans le cyclohexane.

Rendement: 82%; F = 173—176°C.

| | | | | |
|---|---|---|---|---|
| Calc. pour $C_{14}H_{13}ClN_2O$: | C, 64,49; | H, 5,03; | N, 10,74; | Cl, 13,60; |
| Trouvé: | C, 64,45; | H, 5,29; | N, 10,58; | Cl, 13,31. |

RMN H1 [$(CD_3)_2SO$]; δ: 2,28 (d, 3H, $CH_3$-4, $J_{CH3-H-3}$ = 1Hz), 3,91 (s, 3H, $OCH_3$), 4,14 (s, 3H, $NCH_3$), 7,29 (q, 1H, H-7, $J_{7-6}$ = 8,9Hz, $J_{7-9}$ = 2,5Hz), 7,70 (d, 1-H, H-6), 7,91 (d, 1H, H-9), 8 (d, 1H, H3).

Exemple 4

Préparation de chloro-1 hydroxy-8 méthyl-4 5-H pyrido [4,3-b] indole (composé de formule (VI) dans lequel $R'_1$ = H et $R'_2$ = OH).

Le chloro-1 méthyl-4 méthoxy-8 5-H pyrido [4,3-b] indole (200 mg) est chauffé au reflux d'une solution aqueuse concentrée d'acide bromhydrique (7 ml) pendant 1 heure trente. Après refroidissement, le milieu est versé dans l'eau puis neutralisé avec l'ammoniaque. Le précipité formé est filtre puis purifié par chromatographie sur colonne de silice ($CH_2Cl_2/C_2H_5OH$; 98/2) en isolant la fraction dont le Rf est de 0,55 en chromatographie sur couche mince de silice ($CH_2Cl_2/C_2H_5OH$; 9/1).

Rendement: 74,8%; Sublimation au-dessus de 265°C.

Calc. pour $C_{12}H_9ClN_2O$: C, 61,95; H, 3,90; N, 12,04; Cl, 15,24;
Trouvé: C, 62,01; H, 3,83; N, 11.97; Cl, 14,97.

RMN H1 [$(CD_3)_2SO$]; δ: 2,52 (d, 3H, $CH_3$-4), 7,08 (q, 1H, H-7, $J_{7-6}$ = 9Hz, $J_{7-9}$ = 2,4 Hz), 7,49 (d, 1H, H-6), 7,78 (d, 1H, H-9), 8,02 (d, 1H, H-3, $J_{H-3-CH3-4}$ = 1,2Hz), 9,26 (s large, 1H, OH), 11,8 (s large, 1H, NH-5).

Exemple 5

Préparation de chloro-1 diméthyl-4,5 hydroxy-8 5-H pyrido [4,3-b] indole (composé de formule (VI) dans lequel $R'_1$ = —$CH_3$ et $R'_2$ = —OH).

Le chloro-1 diméthyl-4,5 méthoxy-8 5-H pyrido [4,3-b] indole est traité par une solution aqueuse concentrée d'acide bromhydrique selon le mode opératoire de l'exemple 4. Le précipité obtenu après neutralisation par une solution d'ammoniaque est directement recristallisé dans le dioxanne.

Rendement: 65%; F> 260°C.

Calc. pour $C_{13}H_{11}ClN_2O$: C, 63,29; H, 4,49; N, 11,35; Cl, 14,37;
Trouvé: C, 63,48; H, 4,52; N, 11.35; Cl, 13,92.

RMN H1 [$(CD_3)_2SO$]; δ: 2,76 (d, 3H, $CH_3$-4, $J_{CH3-H-3}$ = 1Hz), 4,10 (s, 3H, N-$CH_3$), 7,12 (q, 1H, H-7, $J_{7-6}$ = 9Hz, $J_{7-9}$ = 2,4Hz), 7,58 (d, 1H, H-6), 7,84 (d, 1H, H-9), 7,96 (d, 1H, H-3), 9,34 (s, 1H, OH).

Exemple 6

Préparation de (diéthylamino-3 propylamino)-1 méthyl-4 5-H pyrido [4,3-b] indole et de son bimaléate (SR 95400A) dérivé no 1 (I: $R_1 = R_2$ = H, $R_3 = R_4 = C_2H_5$, $R_5 = R_6 = R_7 = H_1$, n = 1, m = 0).

Le chloro-1 méthyl-4 5-H pyrido [4,3-b] indole (0,5 g) est dissous dans la diéthylamino-3 propylamine (10 ml). Le milieu réactionnel est porté au reflux pendant 48 heures. Puis l'excès d'amine est distillé sous pression réduite. Le résidu est repris dans 10 ml d'eau puis alcalinisé par une solution aqueuse concentrée d'hydroxyde de sodium et enfin extrait par le chlorure de méthylène.

La phase organique est séchée puis concentrée sous pression réduite. Le produit brut ainsi obtenu est purifié par chromatographie sur colonne d'alumine en éluant tout d'abord par le chlorure de méthylène puis avec un mélange chlorure de méthylène-méthanol (98/2).

La base pure ainsi obtenue est dissoute dans une solution acétonique d'acide maléique (au moins 2 équivalents molaires) qui est maintenue à ébullition pendant deux minutes.

Après refroidissement le bimaléate précipité. Il est filtre et séché.

Rendement: 57%; F = 174—178°C avec décomposition.

Calc. pour $C_{27}H_{34}N_4O_8$: C, 59,77; H, 6,32; N, 10,33;
Trouvé: C, 59,40; H, 6,10; N, 10,62;

RMN H1 ($D_2O$); δ: 1,36 (t, 2 × 3H, *$CH_3$*—$CH_2$), 2,03—2,06 (m, 2H, $CH_2$-β), 2,35 (d, 3H, $CH_3$-4), 3,22—3,64 (m, 4 × 2H, *$CH_2$*$CH_3$ + $CH_2$-γ + $CH_2$-α), 6,17 (s, 4H, CH = CH maléate), 7,38 (d, 1H, H-3, $J_{H-3}$-$CH_3$-4 = 1Hz), 7,44—7,59 (m, 3H, H-6 + H-7 + H8), 7,95 (d, 1H, H-9).

Exemple 7

Préparation de (diméthylamino-3 propylamino)-1 méthyl-4 5-H pyrido [4,3-b] indole et de son bimaléate (SR 95403A), dérivé no 2 (I: $R_1 = R_2$ = H, $R_3 = R_4 = CH_3$, $R_5 = R_6 = R_7$ = H, n = 1, m = 0.

Ce composé est préparé selon le mode opératoire de l'exemple 6 au reflux de la diméthylamino-3 propylamine pendant 96 heures.

Après chromatographie, la base est recristallisée dans le toluène.

Rendement: 50%; F = 163—164°C.

Calc. pour $C_{17}H_{22}N_4$: C, 72,30; H, 7,85; N, 19,84;
Trouvé: C, 72,18; H, 7,76; N, 19,77;

Elle est transformée de façon analogue en bimaléate.

F = 212—213°C.

Calc. pour $C_{25}H_{30}N_4O_8$: C, 58,36; H, 5,88; N, 10,89;
Trouvé: C, 58,32; H, 5,74; N, 10,44.

RMN H1 ($D_2O$); δ: 2,1—2,38 (m, 2H, $CH_2$-β), 2,47 (s, 3H, $CH_3$-4), 2,98 (s, 2 × 3H, $N(CH_3)_2$), 3,3—3,5 (m, 2H, $CH_2$-γ), 3,75 (t, 2H, $CH_2$-α), 6,29 (s, 4H, CH = CH— maléate), 7,5—7,8 (m, 4H, H-3 + H-6 + H-7 + H-8), 8,17 (d, 1H, H-9).

Exemple 8

Préparation d'(hydroxyéthylamino-2 éthyl amino)-1 méthyl-4 5-H pyrido [4,3-b] indole et de son

bimaléate (SR 95404A), dérivé no 3 (I: $R_1 = R_2 = H$, $R_3 = H$, $R_4 = CH_2—CH_2—OH$, $R_5 = R_6 = H$, n = m = 0).

Ce composé est préparé selon le mode opératoire de l'exemple 6 dans l'hydroxyéthylamino-2 éthylamine portée à 170°C pendant 16 heures.

La base brute obtenue cristallise directement, elle est recristallisée dans le toluène.

Rendement: 47%; F = 164—166°C.

Calc. pour $C_{16}H_{20}N_4O$: C, 67,58; H, 7,09; N, 19,71;
Trouvé: C, 67,75; H, 7,09; N, 19,46.

RMN H1 [$(CD_3)_2SO$]; δ: 2,38 (s, 3H, $CH_3$-4), 2,69 (t, 2H, NH—$CH_2$$CH_2$—OH), 2,88 (t, 2H, $CH_2$-β), 3,51 (t, 2H, NH—$CH_2$—$CH_2$—OH), 3,61 (t, 2H, $CH_2$-α), 4,45 (s large, 1H, OH), 6,13 (t, 1H-N*H*-1), 7,16—7,6 (m, 3H, H-6 + H-7 + H-8), 7,76 (s, 1H, H-3), 8,2 (d, 1H-H-9), 11,03 (s large, 1H, N*H*-5).

Elle est transformée de façon analogue en bimaléate.

F = 205°C.

Calc. pour $C_{24}H_{28}N_4O_9$: C, 55,80; H, 5,46; N, 10,85;
Trouvé: C, 55,82; H, 5,42; N, 10,67.

Exemple 9

Préparation de (diéthylamino-3 propylamino)-1 méthoxy-8 méthyl-4 5-H pyrido [4,3-b] indole et de son bimaléate (SR 95700A), dérivé no 4 (I: $R_1 = H$, $R_2 = OCH_3$, $R_3 = R_4 = C_2H_5$, $R_5 = R_6 = R_7 = H$, n = 1, m = 0).

Ce composé est préparé par action du chloro-1 méthyl-4 méthoxy-8 5-H pyrido [4,3-b] indole sur la diéthylamino-3 propylamine portée au reflux pendant 96 heures, en suivant le mode opératoire de l'exemple 6.

Après purification par chromatographie, la base est transformée en bimaléate.

Rendement: 62%, F = 176—178°C.

Calc. pour $C_{28}H_{36}N_4O_9$, $H_2O$: C, 56,95; H, 6,44; N, 9,45;
Trouvé: C, 56,72; H, 6,30; N, 9,61.

RMN H1 ($D_2O$); δ: 1,41 (t, 2 × 3H, $CH_3$—$CH_2$), 2,22 (m, 2H, $CH_2$-β), 2,36 (s, 3H, $CH_3$-4), 3,38 (m, 6H, 3 × 2H, $CH_2CH_3$ + $CH_2$-γ), 3,64 (t, 2H, $CH_2$-α), 4,02 (s, 3H, $OCH_3$), 6,16 (s, 4H, CH = CH maléate), 7,17 (q, 1H, H-7, $J_{7-6}$ = 9Hz, $J_{7-9}$ = 2,3Hz), 7,36 (d, 1H, H-6), 7,4 (s large, 1H, H-9), 7,48 (s, 1H, H-3).

Exemple 10

Préparation d'(éthylamino-3 propylamino)-1 méthoxy-8 méthyl-4 5-H pyrido [4,3-b] indole et son bimaléate (SR 95701A), dérivé no 5 (I: $R_1 = H$, $R_2 = OCH_3$, $R_3 = H$, $R_4 = C_2H_5$, $R_5 = R_6 = R_7 = H$, n = 1, m = 0).

Ce composé est préparé selon le mode opératoire de l'exemple 9 au reflux de l'éthylamino-3 propylamine pendant 96 heures.

Après purification par chromatographie, la base est transformée en bimaléate.

Rendement: 80,8%; F = 212—214°C.

Calc. pour $C_{26}H_{32}N_4O_9$; 0,5 $H_2O$: C, 56,42; H, 5,97; N, 10,12;
Trouvé: C, 56,47; H, 6,01; N, 10,02.

RMN H1 ($D_2O$); δ: 1,41 (t, 3H, $CH_3$—$CH_2$), 2,2 (m, 2H, $CH_2$-β), 2,38 (s, 3H, $CH_3$-4), 3,2 (m, 4H, $CH_2$—$CH_3$ + $CH_2$-γ), 3,68 (t, 2H, $CH_2$-α), 4,05 (s, 3H, $OCH_3$), 6,2 (s, 4H, CH = CH maléate), 7,31 (q, 1H, H-7, $J_{7-6}$ = 11Hz, $J_{7-9}$ = 2,5Hz), 7,37 (d, 1H, H-6), 7,44 (m, 1H, H-9), 7,54 (s, 1H, H-3).

Exemple 11

Préparation de (diéthylamino-3 propylamino)-1 diméthyl-4,5 méthoxy-8 5-H pyrido [4,3-b] indole et de son bimaléate (SR 95699A), dérivé no 6 (I: $R_1 = CH_3$, $R_2 = OCH_3$, $R_3 = R_4 = C_2H_5$, $R_5 = R_6 = R_7 = H$, n = 1, m = 0).

Ce composé est préparé par action du chloro-1 diméthyl-4,5 méthoxy-8 5-H pyrido [4,3-b] indole sur la diéthylamino-3 propylamine portée au reflux pendant 44 heures, en suivant le mode opératoire de l'exemple 6.

La base est recristallisée dans l'hexanne.

Rendement: 85,5%; F = 107—109°C.

Calc. pour $C_{21}H_{30}N_4O$: C, 71,15; H, 8,53; N, 15,81;
Trouvé: C, 70,96; H, 8,64; N, 15,57.

RMN H1/$(CD_3)_2SO$/; δ: 0,99 (t, 2 × 3H, $CH_3$—$CH_2$, 1,81 (m, 2H, $CH_2$-β), 2,50—2,54 (m, 3 × 2H, ($CH_2$—$CH_3$) + $CH_2$-γ), 2,62 (d, 3H, $CH_3$-4, $J_{CH3-H-3}$ = 0,7Hz), 3,60 (m, 2H, $CH_2$-α), 3,90 (s, 3H, $OCH_3$), 4,05 (s, 3H, $NCH_3$), 6,35 (t, 1H, NH), 7,09 (q, 1H, H-7, $J_{7-6}$ = 9Hz, $J_{7-9}$ = 2,3Hz), 7,52 (d, 1H, H-6), 7,69 (d, 1H, H-3), 7,74 (d, 1H, H-9).

Puis elle est transformée en bimaléate.

F = 275—276°C.

Calc. pour $C_{29}H_{38}N_4O_9$: C, 59,37; H, 6,53; N, 9,55;
Trouvé: C, 59,20; H, 6,59; N, 9,53.

Exemple 12

Préparation du (diéthylamino-3 propylamino)-1 hydroxy-8 méthyl-4 5-H pyrido [4,3-b] indole de son

bimaléate (SR 95444A), dérivé no 7 et de son bichlorhydrate SR95444B, dérivé no 8 (I: $R_1$ = H, $R_2$ = OH, $R_3$ = $R_4$ = $C_2H_5$, $R_5$ = $R_6$ = $R_7$ = H, n = 1, m = 0).

La diéthylamino-3 propylamine (15 ml) et le chloro-1 hydroxy-8 méthyl-4 5-H pyrido [4,3-b] indole (800 mg) sont chauffés au bain d'huile à 170° pendant 18 h et l'excès d'amine est éliminé sous pression réduite. Le résidu est repris dans 50 ml d'eau, extrait au chlorure de méthylène dans lequel le produit paraît peu soluble et le nouveau résidu obtenu après évaporation du solvant est chromatographié sur colonne d'alumine (35 × 2,2 cm) en éluant avec le chlorure de méthylène pur (300 ml), avec le mélange chlorure de méthylène-éthanol 95/5 v/v (400 ml), puis enfin avec le même mélange, en proportion 9/1 v/v (300 ml). L'évaporation de cette dernière fraction laisse un produit qui est dissous dans l'acétone (50 ml) et versé dans une solution d'acide maléique (1 g) dans 50 ml d'acétone bouillante. Le précipité formé est essoré, agité en suspension dans 50 ml de méthyléthylcétone pendant 48 h, essoré et séché pour donner 740 mg de microcristaux jaunâtres correspondant au bimaléate attendu, hydraté avec une molécule d'eau.

Rendement: 41%; F = 200°C avec décomposition.

Calc. pour $C_{27}H_{34}N_4O_9$; $H_2O$: C, 56,24; H, 6,29; N, 9,72;
Trouvé: C, 56,21; H, 6,27; N, 9,74.

Base libre: recristallise dans le toluène en donnant des microcristaux jaune pâle.

F = 125°C.

Calc. pour $C_{19}H_{26}N_4O$; 0,5 $H_2O$: C, 68,06; H, 8,06; N, 16,72;
Trouve: C, 68,09; H, 8,14; N, 16,55.

RMN H1 [$(CD_3)_2SO$]; δ: 1,0 (t, 2 × 3H, *CH₃*—$CH_2$), 1,80 (m, 2H, $CH_2$-β), 2,30 (s, 3H, CH3-4), 2,4—2,69 (m, 6H, *CH₂*—$CH_3$ + $CH_2$-α), 3,55 -(q, 2H, $CH_2$-α), 6,22 (t, 1H, NH-1), 6,87 (q, 1H, H-7, $J_{7-6}$ = 8,5Hz, $J_{7-9}$ = 2,2Hz), 7,31 (d, 1H, H-6), 7,52 (d, 1H, H-9), 7,66 (s, 1H, H-3), 8,82 (s large, 1H, OH-8), 11,05 (s, 1H, N*H*-5).

La base libre est dissoute dans l'éthanol auquel on ajoute ensuite une solution d'acide chlorhydrique dans l'éther éthylique. Après refroidissement, le chlorhydrate précipité. Il est filtré, lavé à l'éther éthylique, puis recristallisé dans l'éthanol.

Rendement: 72%; F = 208°C.

Calc. pour $C_{19}H_{28}N_4OCl_2$; 2,4 $H_2O$: C, 51,48; H, 7,45; N, 12,64;
Trouvé: C, 51,41; H, 7,05; N, 12,67.

## Example 13

Préparation du (diméthylamino-3 propylamino)-1 hydroxy-8 méthyl-4 5-H pyrido [4,3-b] indole SR 95443 (dérivé no 9) et de son bimaléate SR 95443A (dérivé no 10) (I: $R_1$ = H, $R_2$ = OH, $R_3$ = $R_4$ = $CH_3$, $R_5$ = $R_6$ = $R_7$ = H, n = 1, m = 0).

Ce composé est préparé selon le mode opératoire de l'exemple 12 en présence de diméthylamino-3 propylamine, portée à 180°C en autoclave pendant 24 heures. Après purification par chromatographie, la base est recristallisée dans le toluène.

Rendement: 78%; F = 235—245°C avec décomposition.

Calc. pour $C_{17}H_{22}N_4O$: C, 68,43; H, 7,43; N, 18,78;
Trouvé: C, 68,42; H, 7,51; N, 18,54.

RMN H1 [$(CD_3)_2SO$]; δ: 1,83 (m, 2H, $CH_2$-β), 2,26 (s, 2 × 3H, $N(CH_3)_2$), 2,33 (d, 3H, $CH_3$-4), 2,38—2,57 (m, 2H, $CH_2$-γ), 3,59 (m, 2H, $CH_2$-α), 6,44 (t, 1H, N*H*-1), 6,90 (d, 1H, H-7, $J_{7-6}$ = 8,5Hz, $J_{7-9}$ = 2Hz), 7,33 (d, 1H, H-6), 7,52 (d, 1H, H-9), 7,68 (d, 1H, H-3, JH-3-$CH_3$-4 = 1Hz), 8,82 (s large, 1H, OH-8), 11,06 (s large, 1H, N*H*-5).

Son bimaléate est préparé de façon habituelle.

F = 210—220°C avec décomposition.

Calc. pour $C_{17}H_{22}N_4O$; 2 $C_4H_4O_4$; 0,5 $H_2O$: C, 55,65; H, 5,75; N, 10,38;
Trouvé: C, 55,50; H, 5,74; N, 10,39.

## Exemple 14

Préparation du (diéthylamino-3 propylamino)-1 diméthyl-4,5 hydroxy-8 5-H pyrido [4,3-b] indole, de son bimaléate SR 95646A (dérivé no 11) et de so bichlorhydrate SR 95646B (dérivé no 12) (I: $R_1$ = $CH_3$, $R_2$ = OH, $R_3$ = $R_4$ = $C_2H_5$, $R_5$ = $R_6$ = $R_7$ = H, n = 1, m = 0).

Ce composé est préparé selon le mode opératoire de l'exemple 12 au reflux de la diéthylamino-3 propylamine pendant 72 heures.

La base est recristallisée dans l'acétate d'éthyle.

Rendement: 49%; F = 187—189°C.

Calc. pour $C_{20}H_{28}N_4O$: C, 70,55; H, 8,29; N, 16,46;
Trouvé: C, 70,30; H, 8,21; N, 16,17.

RMN H1 [$(CD_3)_2SO$]; δ: 1,0 (t, 2 × 3H, *CH₃*—$CH_2$), 1,81 (m, 2H, $CH_2$-β), 2,52—2,57 (m, 3 × 2H, *CH₂*—$CH_3$ + $CH_2$-γ), 2,60 (d, 3H, $CH_3$-4), 3,58 (q, 2H, $CH_2$-α), 4,02 (s, 3H, $NCH_3$), 6,28 (m, 1H, NH-1), 6,95 (q, 1H, H-7, $J_{7-6}$ = 8,7Hz, $J_{7-9}$ = 2,2Hz), 7,40 (d, 1H, H-6), 7,56 (d, 1H, H-9), 7,66 (d, 1H, H-3, JH-3-$CH_3$-4 = 0,7Hz), 8,92 (s large, 1H, OH).

Elle est ensuite transformée en bimaléate:

F = 176—178°C.

Calc. pour $C_{20}H_{28}N_4O$, $C_8H_8O_8$: C, 58,73; H, 6,34; N, 9,79;
Trouvé: C, 58,51; H, 6,61; N, 9,84.

Au départ de la base libre, le chlorhydrate est préparé comme dans l'exemple 12.
Rendement: 85%; F = 200°C.
Calc. pour $C_{20}H_{30}N_4OCl_2$; 1,25 $H_2O$: C, 55,10; H, 7,51; N, 12,85;
Trouvé: C, 55,16; H, 7,58; N, 12,73.

Exemple 15

Préparation du bichlorhydrate de diméthyl-4,5 (diméthylamino-3 propylamino)-1 méthoxy-8 5-H pyrido [4,3-b] indole SR 26125A (dérivé no 13) (I: $R_1$ = $CH_3$, $R_2$ = $OCH_3$, $R_3$ = $R_4$ = $CH_3$, $R_5$ = $R_6$ = $R_7$ = H, n = 1, m = 0).

Ce composé est préparé selon le mode opératoire de l'exemple 6 à partir de chloro-1 diméthyl-4,5 méthoxy-8 pyrido [4,3-b] indole en présence de diméthylamino-3 propylamine portée à 200°C en autoclave pendant 4 heures. Après purification par chromatographie (éluant: méthanol/triéthylamine; 9/1) on isole la base pure.

Rendement: 61%; F = 108°C.
La base est transformée en chlorhydrate comme dans l'exemple 12.
Rendement: 92%; F = 200°C.
Calc. pour $C_{19}H_{28}N_4OCl_2$; 2,25 $H_2O$: C, 51,89; H, 7,45; N, 12,73;
Trouvé: C, 51,93; H, 7,34; N, 12,66.

RMN H1 [$D_2O$ + $CF_3CO_2D$]; δ: 2,25—2,50 (m, 2H, $CH_2$-β), 2,56 (s, 3H, $CH_3$-4), 3,12 (s, 2 × 3H, N—$CH_3$), 3,40—3,59 (m, 2H, $CH_2$-γ), 3,71—3,90 (m, 2H, $CH_2$-α), 3,78 (s, 3H, $CH_3$-5), 4,03 (s, 3H, O—$CH_3$), 7,16 (q, 1H, H-7, $J_{7\text{-}6}$ = 10Hz, $J_{7\text{-}9}$ = 2,5Hz), 7,36 (d, 1H, H-6), 7,47 (d, 1H, H-9), 7,50 (s, 1H, H-3).

Exemple 16

Préparation du diméthyl-4,5 (diméthylamino-3 propylamino)-1 hydroxy-8 5-H pyrido [4,3-b] indole, de son bimaléate SR 95647A (dérivé no 14) et de son bichlorhydrate SR 95647B (dérivé no 15) (I: $R_1$ = $CH_3$, $R_2$ = OH, $R_3$ = $R_4$ = $CH_3$, $R_5$ = $R_6$ = $R_7$ = H, n = 1, m = 0).

Ce composé est préparé selon le mode opératoire de l'exemple 12 au reflux de la diméthylamino-3 propylamine pendant 14 jours.

La base est recristallisée dans l'acétate d'éthyle.

Ce composé peut également être préparé à partir du dérivé no 13 selon le mode opératoire de l'exemple 20.

Rendement: 50,5%; Sublimation au-dessus de 260°C.
Calc. pour $C_{18}H_{24}N_4O$: C, 69,20; H, 7,75; N, 17,94;
Trouvé: C, 69,17; H, 7,79; N, 17,65.

RMN H1 [$(CD_3)_2SO$]; δ: 1,83 (m, 2H, $CH_2$-β), 2,25 (s, 2 × 3H, $N(CH_3)_2$), 2,45 (m, 2H, $CH_2$-γ), 2,61 (s, 3H, $CH_3$-4), 3,6 (m, 2H, $CH_2$-α), 4,02 (s, 3H, $NCH_3$), 6,51 (s, 1H, NH), 6,97 (q, 1H, H-7, $J_{7\text{-}6}$ = 8,8Hz, $J_{7\text{-}9}$ = 2,4Hz), 7,41 (d, 1H, H-6), 7,54 (d, 1H, H-9), 7,67 (s, 1H, H-3), 8,94 (s large, 1H, OH).

Elle est transformée en bimaléate.
F = 208—210°C.
Calc. pour $C_{18}H_{24}N_4O$, $C_8H_8O_8$, 1/2$H_20$: C, 56,42; H, 5,96; N, 10,12;
Trouvé: C, 56,52; H, 6,15; N, 10,53.
Le bichlorhydrate est préparé à partir de la base comme dans l'exemple 13.
Rendement: 89%; F = 258°C.
Calc. pour $C_{18}H_{26}N_4OCl_2$; 1,25 $H_20$: C, 53,01; H, 6,73; N, 13,73;
Trouvé: C, 52,85; H, 7,06; N, 13,53.

Exemple 17

Préparation de bichlorhydrate de (diéthylamino-3 propylamino)-1 diméthyl-4,5 5-H pyrido [4,3-b] indole, SR 26021A (dérivé no 16) (I: $R_1$ = $CH_3$, $R_2$ = H, $R_3$ = $R_4$ = $C_2H_5$, $R_5$ = $R_6$ = $R_7$ = H, n = 1, m = 0).

A) Chloro-1 diméthyl-4,5 5-H pyrido [4,3-b] indole.

A une solution de chloro-1 méthyl-4 5-H pyrido [4,3-b] indole (3,3 g; 0,015 mole), prépare selon le mode opératoire de l'exemple 1, dans 100 ml de N,N-diméthylformamide sec, on ajoute 11,5 g (0,08 mole) de carbonate de potassium puis 4,7 g (0,033 mole) d'iodure de méthyle. Sous atmosphère d'azote le milieu est agité pendant 5 heures à température ordinaire. Après concentration sous pression réduite à 25°C, on dissout le produit obtenu dans une solution aqueuse N d'acide chlorhydrique.

Puis on neutralise la solution aqueuse par l'ammoniaque. Le précipité obtenu est filtré, lavé à l'eau puis à l'acetone. Le dérivé chloré ainsi obtenu est purifiée par chromatographie sur silice (éluant: acétate d'éthyle).

Rendement: 59%; F = 214°C.
Calc. pour $C_{13}H_{11}N_2Cl$: C, 67,67; H, 4,81; N, 12,14; Cl, 15,37;
Trouvé: C, 67,59; H, 4,73; N, 11,93; Cl, 15,77.

B) Bichlorhydrate de (diéthylamino-3 propylamino)-1 diméthyl-4,5 5-pyrido [4,3-b] indole.

Ce composé est préparé selon le mode opératoire de l'exemple 6 à partir du dérivé chloré obtenu à l'étape précédente en présence de diéthylamino-3 propylamine portée à 200°C en autoclave pendant 6 heures. Après purification par chromatographie (éluant: méthanol/triéthylamine; 9/1), on isole la base pure sous forme d'huile. Elle est transformée en chlorhydrate comme dans l'exemple 12.

Rendement: 39%; F = 222°C.

Calc. pour $C_{20}H_{30}N_4Cl_2$; 2,75 $H_2O$: C, 53,75; H, 8,01; N, 12,53; Cl, 15,86;
Trouvé: C, 53,56; H, 8,06; N, 12,33; Cl, 16,03.

Exemple 18

Préparation du bichlorhydrate de diméthyl-4,5 (diméthylamino-3 propylamino)-1 5-H pyrido [4,3-b] indole, SR 26022A (dérivé no 17) (I: $R_1$ = $CH_3$, $R_2$ = H, $R_3$ = $R_4$ = $CH_3$, $R_5$ = $R_6$ = $R_7$ = H, n = 1, m = 0).

Ce composé est préparé selon le mode opératoire de l'exemple 17 en présence de diméthylamino-3 propylamine.

Rendement: 63%; F = 250°C.

Calc. pour $C_{18}H_{26}N_4Cl_2$; 1,5 $H_2O$: C, 54,55; H, 7,37; N, 14,13;
Trouvé: C, 54,60; H, 7,50; N, 13,98.

Exemple 19

Préparation du bichlorhydrate de (diéthylamino-2 éthylamino)-1 diméthyl-4,5 méthoxy-8 5-H pyrido [4,3-b] indole, SR 26152A (dérivé no 18) (I: $R_1$ = $CH_3$, $R_2$ = $OCH_3$, $R_3$ = $R_4$ = $C_2H_5$, $R_5$ = $R_6$ = H, n = m = 0).

Ce composé est prépare selon le mode opératoire de l'exemple 6 à partir du chloro-1 diméthyl-4,5 méthoxy-8 5-H pyrido [4,3-b] indole obtenu dans l'exemple 3 en présence de diéthylamino-2 éthylamine portée à 200—210°C en autoclave pendant 4 heures. La base, après purification par chromatographie (éluant: méthanol) est transformée chlorhydrate selon le mode opératoire de l'exemple 12.

Rendement: 83%; F = 236°C.

Calc. pour $C_{20}H_{30}N_4OCl_2$; 0,5 $H_2O$: C, 56,80; H, 7,40; N, 13,26; Cl, 16,80;
Trouvé: C, 57,00; H, 7,24; N, 13,27; Cl, 16,80.

RMN H1 ($D_2O$); δ: 1,47 (t, 2 × 3H, $CH_2$—$CH_3$), 2,44 (d, 3H, $CH_3$-4, $JCH_3$-4-H3 = 0,9Hz), 3,34—3,62 (m, 3 × 2H, $CH_2$—$CH_3$ et $CH_2$-β), 3,50 (s, 3H, $CH_3$-5), 3,90—4,09 (t, 2H, $CH_2$-α), 3,99 (s, 3H, $OCH_3$), 6,98 (q, 1H, H7, $J_{7-6}$ = 8,9Hz, $J_{7-9}$ = 2,2Hz), 7,14 (d, 1H, H-6), 7,34 (d, 1H, H-9), 7,45 (d, 1H, H-3).

Exemple 20

Préparation du bichlorhydrate de (diéthylamino-2 éthylamino)-1 diméthyl-4,5 hydroxy-8 5-H pyrido [4,3-b] indole, SR 26153A (dérivé no 19) (I: $R_1$ = $CH_3$, $R_2$ = OH, $R_3$ = $R_4$ = $C_2H_5$, $R_5$ = $R_6$ = H, n = m = 0).

Dans 30 ml d'une solution aqueuse à 48% d'acide bromhydrique, on ajoute 3 g de bichlorhydrate de (diéthylamino-2 éthyl)amino-1 diméthyl-4,5 méthoxy-8 5-H pyrido [4,3-b] indole obtenu dans l'exemple 19 et l'on porte le milieu à reflux pendant 3 heures. La solution est ensuite concentrée sous pression réduite. Le produit brut obtenu est lavé à l'acétone puis purifié par chromatographie sur colonne de silice (éluant: méthanol/triéthylamine; 9/1). La base pure est ensuite transformée en chlorhydrate selon le mode opératoire de l'exemple 12. On isole ainsi 2,1 g de chlorhydrate attendu.

Rendement: 75%; F = 260°C.

Calc. pour $C_{19}H_{28}N_4OCl_2$; 1 $H_2O$: C, 54,67; H, 7,19; N, 13,42; Cl, 17,00;
Trouvé: C, 54,40; H, 7,29; N, 13,15; Cl, 16,71.

RMN H1 ($D_2O$); δ: 1,47 (t, 2 × 3H, $CH_2CH_3$), 2,40 (d, 3H, $CH_3$-4, $JCH_3$-4-H3 = 0,9Hz), 3,34—3,62 (m, 3 × 2H, $CH_2$—$CH_3$ et $CH_2$-β), 3,47 (s, 3H, $CH_3$-5), 3,84—4,03 (m, 2H, $CH_2$-α), 6,86 (q, 1H, H-7, $J_{7-6}$ = 8,9Hz, $J_{7-9}$ = 2,2Hz), 7,02 (d, 1H, H-6), 7,19 (d, 1H, H-9), 7,33 (d, 1H, H-3).

Exemple 21

Préparation du bichlorhydrate de diméthyl-4,5 (diméthylamino-3 méthyl-2 propylamino)-1 méthoxy-8 5-H pyrido [4,3-b] indole, SR 26165A (dérivé no 20) (I: $R_1$ = $CH_3$, $R_2$ = $OCH_3$, $R_3$ = $R_4$ = $CH_3$, $R_5$ = H, $R_6$ = $CH_3$, $R_7$ = H, n = 1, m = 0).

Ce composé est préparé selon le mode opératoire de l'exemple 19 en présence de diméthylamino-3 méthyl-2 propylamine.

Rendement: 66%; F = 220°C.

Calc. pour $C_{20}H_{30}N_4OCl_2$; 1,75 $H_2O$: C, 53,99; H, 7,59; N, 12,60;
Trouvé: C, 53,86; H, 7,67; N, 12,34.

RMN H1 ($D_2O$); δ: 1,41 (d, 3H, CH—$CH_3$), 2,53 (s, 3H, $CH_3$-4), 2,61—2,93 (m, 1H, CH), 3,22 (s, 2 × 3H, N—$CH_3$), 3,37—3,81 (m, 2 × 2H, $CH_2$-γ et $CH_2$-α), 3,52 (s, 3H, $CH_2$-5), 4,09 (s, 3H, $OCH_3$), 7,16 (q, 1H, H-7, $J_{7-6}$ = 9Hz, $J_{7-9}$ = 2Hz), 7,34 (d, 1H, H-6), 7,53 (d, 1H, H-9), 7,58 (s, 1H, H-3).

Exemple 22

Préparation du bichlorhydrate de diméthyl-4,5 (diméthylamino-3 méthyl-2 propylamino)-1 hydroxy-8 5-H pyrido [4,3-b] indole, SR 26166A (dérivé no 21) (I: $R_1$ = $CH_3$, $R_2$ = OH, $R_3$ = $R_4$ = $CH_3$, $R_5$ = H, $R_6$ = $CH_3$, $R_7$ = H, n = 1, m = 0).

Ce composé est préparé selon le mode opératoire de l'exemple 20 à partir de diméthyl-4,5

(diméthylamine-3 méthyl-2 propylamino)-1 méthoxy-8 5-H pyrido [4,3-b] indole.

Rendement: 67%; F = 236°C.

Calc. pour $C_{19}H_{28}N_4OCl_2$; 2,25 $H_2O$: C, 51,87; H, 7,44; N, 12,73;

Trouvé: C, 52,07; H, 6,97; N, 12,71.

RMN H1 ($D_2O$); δ: 1,23 (d, 3H, CH—*H*$_3$), 2,38 (d, 3H, $CH_3$-4, JH3-$CH_3$-4 = 0,2Hz), 2,53—2,71 (m, 1H, CH), 3,04 (s, 2 × 3H, N—$CH_3$), 3,23 (d, 2H, $CH_2$-γ), 3,49—3,60 (q, 2H, $CH_2$-α), 3,64 (s, 3H, $CH_3$-5), 7,05 (q, 1H, H-7, $J_{7\text{-}6}$ = 9Hz, $J_{7\text{-}9}$ = 2Hz), 7,21 (d, 1H, H-6), 7,25 (d, 1H, H-9), 7,34 (d, 1H, H-3).

Exemple 23

Préparation du bichlorhydrate de (diéthylamino-4 butylamino)-1 diméthyl-4,5 méthoxy-8 5-H pyrido [4,3-b] indole, SR 26180A (dérivé no 22) (I: $R_1$ = $CH_3$, $R_2$ = $OCH_3$, $R_3$ = $R_4$ = $C_2H_5$, $R_5$ = $R_6$ = $R_7$ = $R_8$ = H, n = m = 1).

Ce composé est préparé selon le mode opératoire de l'exemple 19 en présence de diéthylamino-4 butylamine. Le chlorhydrate est obtenu en milieu isopropanol.

Rendement: 26%; F = 232°C.

Calc. pour $C_{22}H_{34}N_4OCl_2$; 0,25 $H_2O$: C, 59,25; H, 7,80; N, 12,56;

Trouvé: C, 59,08; H, 7,87; N, 12,45.

RMN H1 ($D_2O$); δ: 1,28 (t, 2 × 3H, $CH_2$—*CH*$_3$), 1,72—1,97 (m, 2 × 2H, $CH_2$-β et $CH_2$-γ), 2,47 (s, 3H, $CH_3$-4), 3,09—3,36 (m, 3 × 2H, *CH*$_2$—$CH_3$, $CH_2$-δ), 3,50—3,67 (m, 2H, $CH_2$-α), 3,81 (s, 3H, $CH_3$-5), 3,97 (s, 3H, $OCH_3$), 7,20 (q, 1H, H-7, $J_7$ = 9Hz, $J_{7\text{-}9}$ = 2Hz), 7,34 (d, 1H, H-6), 7,37 (d, 1H, H-9), 7,47 (s, 1H, H-3).

Exemple 24

Préparation du bichlorhydrate de (diéthylamino-4 butylamino)-1 diméthyl-4,5 hydroxy-8 5-H pyrido [4,3-b] indole, SR 26181A (dérivé no 23) (I: $R_1$ = —$CH_3$, $R_2$ = OH, $R_3$ = $R_4$ = $C_2H_5$, $R_5$ = $R_6$ = $R_7$ = $R_8$ = H, n = m = 1).

Ce composé est préparé selon le mode opératoire de l'exemple 20 à partir de (diéthylamino-4 butylamino)-1 diméthyl-4,5 méthoxy-8 5-H pyrido [4,3-b] indole. Le chlorhydrate est obtenu en milieu isopropanol.

Rendement: 44%; F = 180°C.

Calc. pour $C_{21}H_{32}N_4OCl_2$; 2,75 $H_2O$: C, 52,90; H, 7,93; N, 11,75;

Trouvé: C, 53,12; H, 7,78; N, 11,59.

RMN H1 ($D_2O$); δ: 1,28 (t, 2 × 3H, $CH_2$—*CH*$_3$), 1,69—1,91 (m, 2 × 2H, $CH_2$-β et $CH_2$-γ), 2,28 (s, 3H, $CH_3$-4), 3,08—3,41 (4 × 2H, *CH*$_2$—$CH_3$, $CH_2$-δ et $CH_2$-α), 3,47 (s, 3H, $CH_3$-5), 6,86 (q, 1H, H-7, $J_{7\text{-}6}$ = 9Hz, $J_{7\text{-}9}$R = 2Hz), 7,00 (d, 1H, H-6), 7,12 (d, 1H, H-9), 7,19 (s, 1H, H-3).

Exemple 25

Préparation de (diéthylamino-3 hydroxy-2 propylamino)-1 diméthyl-4,5 hydroxy-8 5-H pyrido [4,3-b] indole, SR 95964 (dérivé no 24) et de son bichlorhydrate, SR 95964A (dérivé no 25) (I: $R_1$ = $CH_3$, $R_2$ = OH, $R_3$ = $R_4$ = $C_2H_5$, $R_5$ = H, $R_6$ = OH, $R_7$ = H, n = 1, m = 0).

Ce composé est préparé selon le mode opératoire de l'exemple 12 à partir de diméthyl-4,5 chloro-1 hydroxy-8 5-H pyrido [4,3-b] indole obtenu selon l'exemple 5 en présence de diéthylamino-3 hydroxy-2 propylamine. La base est simplement purifiée par recristallisation dans le toluène.

Rendement: 59%; F = 180—181°C.

Calc. pour $C_{20}H_{28}N_4O_2$; 0,2 $H_2O$: C, 66,74; H, 7,90; N, 15,57;

Trouvé: C, 66,74; H, 7,94; N, 15,40.

RMN H1 [$(CD_3)_2SO$]; δ: 1,00 (t, 2 × 3H, $CH_2$—*CH*$_3$), 2,53 (m, 2 × 2H, *CH*$_2$—$CH_3$), 2,61 (d, 3H, $CH_3$-4, J$CH_3$-4-H3 = 0,4Hz), 3,63 (m, 4H, 2 × 2H, $CH_2$-α, $CH_2$-γ), 3,84 (m, 1H, *CH*—OH), 4,03 (s, 3H, $CH_3$-5), 5,24 (s, 1H, —OH), 6,10 (t, 1H, NH), 6,97 (q, 1H, H-7, $J_{7\text{-}6}$ = 8,4Hz, $J_{7\text{-}9}$ = 1,8Hz), 7,38—7,50 (m, 2H, H-6 + H-9), 7,64 (d, 1H, H-3), 9,02 (s, 1H, OH-8).

Le chlorhydrate est préparé selon la méthode décrite dans l'exemple 12.

Rendement: 92%; F = 250°C.

Calc. pour $C_{20}H_{30}N_4O_2Cl_2$; 0,75 $H_2O$: C, 54,29; H, 7,17; N, 12,66;

Trouvé: C, 54,16; H, 7,06; N, 16,61.

Les résultats des études pharmacologique et toxicologique rapportés ci-après, ont mis en évidence les intéressantes propriétés des dérivés de l'invention, tant sur le plan de la toxicité et de la tolérance que sur le plan de leur activité antitumorale.

L'invention a donc encore pour objet, un médicament présentant, en particulier, une activité antitumorale, caractérisé en ce qu'il contient às titre de principe actif un dérivé de la formule (I) et de ses formes tautomères ainsi que les sels d'addition avec les acides minéraux ou organiques pharmaceutiquement acceptables.

Etude Toxicologique

Les composés de l'invention bénéficient d'une bonne tolérance et d'une faible toxicité.

Ainsi l'administration, à des souris hydrides $CDF_1$ saines, en une injection unique par voie intrapéritonéale, des composés à tester a permis de constater que le produit est parfaitement toléré à dose

élevée puisque le taux de survivants, parmi les animaux d'expérience, était de 100 p. 100 treize jours après l'administration.

Les résultats sont rassemblés dans le tableau ci-après.

| dérivé | dose mg/kg | Pourcentage de survivants treize jours après l'administration. |
|---|---|---|
| N° 1 | 100 | 100 |
| N° 4 | 50 | 100 |
| N° 7 | 50 | 100 |
| N° 10 | 100 | 100 |
| N° 11 | 50 | 100 |
| N° 14 | 25 | 100 |

En outre l'administration, à des souris hybrides $CDF_1$ femelles saines en une injection unique par voie intraveineuse d'une durée de 4 minutes à l'aide d'un perfuseur, des composés à tester en solution dans le sérum physiologique à raison de 0,1 ml de solution par 10 g de poids des animaux a permis de constater que les composés sont également bien tolérés à dose élevée dans ces conditions.

Les résultats sont rassemblés dans le tableau ci-après.

| Dérivé | Dose mg/kg | Pourcentage de survivants huit jours après l'administration |
|---|---|---|
| N° 7 | 25<br>30 | 100<br>100 |
| N° 10 | 25<br>40 | 100<br>40 |
| N° 11 | 40<br>50 | 100<br>100 |
| N° 14 | 50 | 100 |

La toxicité a été aussi évaluée en suivant, tout au long des essais, la mortalité et l'évolution pondérale des animaux d'expérience. On a constate ainsi que cela est rapporté plus loin, qu'aux doses efficaces, les composés de l'invention étaient bien tolérés et que l'indice thérapeutique était nettement favorable.

Etude Pharmacologique

Elle a été réalisée in vitro et in vivo.

1) In vitro: (PAOLETTI et al, Chem. Biol. Interaction, 1979, *25*, 45—58).

L'activité cytotoxique des composés de l'invention a été évaluée par des essais vitro. Ces essais consistent à ajouter des concentrations croissantes du composé à tester à une culture cellulaire de la lignée tumorale leucémie L 1210 en phase exponentielle de croissance. Les cellules sont incubées à 37°C dans une étuve à $CO_2$ et comptées toutes les 24 heures. le calcul de 1'I D 50 (concentration du produit exprimée en micromole $1^{-1}$ qui inhibe la prolifération cellulaire à 50 p.100) est effectué après 48 heures de contact.

Ainsi déterminées, les I D 50 des composés de l'invention sont rassemblés dans le tableau suivant:

| Dérivé | I D 50 Micromole $l^{-1}$ |
|---|---|
| 1 | 0,20 |
| 2 | 0,16 |
| 3 | 1,14 |
| 4 | 0,30 |
| 5 | 1,20 |
| 6 | 0,10 |
| 7 | 0,06 |
| 8 | 0,08 |
| 9 | 0,09 |
| 11 | 0,03 |
| 12 | 0,035 |
| 13 | 0,13 |
| 14 | 0,014 |
| 15 | 0,01 |
| 16 | 0,72 |
| 17 | 0,35 |
| 18 | 1,20 |
| 19 | 0,15 |
| 20 | 1,00 |
| 21 | 0,01 |
| 22 | 1,30 |
| 23 | 0,31 |
| 25 | 0,063 |

2) In vivo: (GERAN et al, Cancer Chemother., 1972, *2*, 07—57):

L'activité antitumorale a été testée in vivo sur différentes tumeurs inoculées selon des voies variables en suivant le protocole général suivant:

Toutes les souris mâles ou femelles sont inoculées au jour J0 par une quantité déterminée de cellules tumorales viables pour les tumeurs ascitiques ou par un fragment tumoral déterminé pour les tumeurs solides.

Elles sont ensuite réparties en différents lots. Chaque lot, correspondant aux animaux traités par le produit à tester, est composé de 10 souris. Le produit à tester est dissous dans l'eau distillée et il est administré n fois (Jx — Jy), n, x et y variables selon la protocole choisi, à raison de 0,1 ml de solution par 10 g de poids de souris traitées et à des doses spécifiées pour chaque protocole.

Le lot "contrôle négatif", correspondant aux animaux qui ne sont pas traités par un produit, comprend $2\sqrt{N}$ animaux, N étant égal ou supérieur au nombre total des souris traitées par un produit dans la même expérience.

Enfin, les essais peuvent être également conduits parallèlements à l'étude d'un lot appelé "contrôle positif" de 10 animaux qui est traité par une molécule, connue pour son activité antitumorale et variable selon le protocole choisi, dans les mêmes conditions que les lots traités par le produit à tester.

L'activité antitumorale est évaluée, dans le cas des tumeurs ascitiques, en considérant l'augmentation du temps de survie des animaux traités par rapport à celui des animaux du lot "contrôle négatif" selon la formule:

$$T/C = \frac{\text{Jour médian de survie des animaux du lot traité à une dose donnée de produit}}{\text{Jour médian de survie des animaux du lot "contrôle négatif"}} \times 100$$

Le critère d'activité est variable selon le protocole.

De plus, un produit est considéré comme toxique à la dose étudiée lorsque cette valeur T/C est égale ou inférieure à 85%. En outre, un produit est également considéré comme toxique à la dose étudiée lorsque la variation pondérale, exprimée en grammes et calculée par la formule:

"Poids moyen du lot mesuré au jour — Poids moyen de même lot mesuré au jour 1"

est égale ou inférieure à — 4 g.

L'activité antitumorale est évaluée, dans le cas des tumeurs solides en considérant la réduction du volume tumoral moyen des animaux traités par rapport à celui des animaux du lot "contrôle négatif" selon la formule:

$$T/C = \frac{\text{Volume tumoral moyen des animaux du lot traité à une dose donnée de produit et au jour J}}{\text{Volume tumoral moyen du lot "contrôle négatif" mesuré au même jour}} \times 100$$

Une valeur de T/C inférieure à 42% traduit une activité du produit à la dose étudiée.

a) Test de la leucémie P 388; IP/IP, J1—5 (GERAN et al, Cancer Chemother., 1972, *2*, 07—57).

Les essais sont effectués avec des souris hybrides CDF 1 inoculées par voie intrapéritonéale avec $10^6$ cellules leucémiques de la lignée P 388. Le composé à tester est administré pendant 5 jours successifs (J1—J5) par voie intrapéritoneale. Le contrôle positif du test est 5-Fluorouracile qui est administré dans les mêmes conditions.

Le nombre d'animaux survivants est evalués au jour J30. Un composé est considéré comme étant actif lorsque le rapport T/C est supérieur à 127%.

Les résultats obtenus sont consignés dans le tableau no 1 ci-après.

TABLEAU N° 1

| Produit testé | Dose (mg/kg) | Variation pondérale (g) | Jour médian de survie | T/C % | Nombre de survivants |
|---|---|---|---|---|---|
| Dérivé N°1 | 10<br>50 | -0,9<br>-2,6 | 15,7<br>21,0 | 130<br>174 | 0/10<br>0/10 |
| Contrôle positif : 5-Fluorouracile | 20 | +1,3 | 20,1 | 166 | 0/10 |
| Contrôle négatif | - | -0,8 | 12,1 | - | 0/36 |
| Dérivé N°4 | 5<br>10<br>20<br>40<br>80 | +1,1<br>+0,7<br>-0,9<br>-3,4<br>-4,8 | 14,0<br>17,0<br>17,25<br>20,3<br>6,6 | 121<br>147<br>149<br>176<br>57 | 0/10<br>0/10<br>0/10<br>0/10<br>0/10 |
| Contrôle positif : 5-Fluorouracile | 20 | +0,3 | 18,9 | 164 | 0/10 |
| Contrôle négatif | - | +1,0 | 11,55 | - | 0/30 |
| Dérivé N°7 | 0,625<br>1,25<br>2,5<br>5<br>10<br>15<br>20<br>30 | +0,3<br>+0,3<br>+0,1<br>+0,3<br>-0,2<br>+1,6<br>-1,1<br>0,0 | 14,3<br>17,25<br>18,25<br>19,25<br>20,9<br>23,55<br>27,0<br>9,25 | 127<br>153<br>162<br>170<br>185<br>208<br>238<br>81 | 0/10<br>0/10<br>0/10<br>0/10<br>0/10<br>3/10<br>1/10<br>0/10 |
| Contrôle positif : 5-Fluorouracile | 20 | +0,6 | 19 | 168 | 0/10 |
| Contrôle négatif | - | +4,0 | 11,3 | - | 0/10 |

(Suite Tab. N° 1)

| Produit testé | Dose (mg/kg) | Variation pondérale (g) | Jour médian de survie | T/C % | Nombre de survivants |
|---|---|---|---|---|---|
| Dérivé N°8 | 0,625 | -0,5 | 15,1 | 136 | 0/10 |
| | 1,25 | +0,6 | 15,7 | 141 | 0/10 |
| | 2,5 | -1,2 | 16,1 | 147 | 0/10 |
| | 5 | -0,8 | 19,4 | 175 | 0/10 |
| | 10 | -1,1 | 12,0 | 108 | 0/10 |
| Contrôle positif : 5-Fluorouracile | 20 | -0,8 | 17,75 | 160 | 0/10 |
| Contrôle négatif | - | +0,2 | 11,1 | - | 0/20 |
| Dérivé N° 10 | 0,625 | +4,1 | 15,75 | 139 | 0/10 |
| | 1,25 | +3,3 | 17,0 | 150 | 0/10 |
| | 2,5 | +3,2 | 16,4 | 145 | 0/10 |
| | 5 | +2,0 | 21,0 | 186 | 1/10 |
| | 10 | +1,8 | 16,75 | 148 | 0/10 |
| | 20 | -0,4 | 7,8 | 69 | 0/10 |
| Contrôle positif : 5-Fluorouracile | 20 | +3,7 | 16,7 | 147 | 0/10 |
| Contrôle négatif | - | +4,0 | 11,3 | - | 0/25 |
| Dérivé N° 11 | 2,5 | +1,2 | 17,25 | 149 | 0/10 |
| | 5 | +0,7 | 19,25 | 166 | 0/10 |
| | 10 | +0,2 | 23,75 | 205 | 2/10 |
| | 20 | -2,8 | 7,4 | 64 | 0/10 |
| Contrôle positif 5-Fluorouracile | 20 | +1,3 | 20,1 | 173 | 0/10 |
| Contrôle négatif | - | +1,0 | 11,56 | - | 0/20 |
| Dérivé N° 12 | 1,25 | -0,5 | 16,7 | 152 | 1/10 |
| | 2,5 | -1,2 | 18,0 | 164 | 0/10 |
| | 5 | -1,9 | 19,00 | 173 | 2/10 |
| | 10 | -3,5 | 19,3 | 176 | 0/10 |
| | 20 | -5,9 | 9,7 | 88 | 0/10 |
| Contrôle positif : 5-Fluorouracile | 20 | -0,1 | 18,9 | 171 | 0/10 |
| Contrôle négatif | - | -0,95 | 11,0 | - | 0/20 |

| Produit testé | Dose (mg/kg) | Variation pondérale (g) | Jour médian de survie | T/C % | Nombre de survivants |
|---|---|---|---|---|---|
| Dérivé N° 13 | 5<br>10<br>20<br>40 | +1,0<br>+1,2<br>+0,4<br>-2,5 | 14,1<br>16,8<br>15,8<br>8,4 | 134<br>160<br>150<br>80 | 0/10<br>0/10<br>0/10<br>0/10 |
| Contrôle négatif | - | +2,1 | 10,5 | - | 0/25 |
| Dérivé N° 14 | 2,5<br>5<br>10<br>20 | +0,9<br>-1,0<br>-1,7<br>-4,4 | 19,25<br>22<br>11,75<br>7,4 | 166<br>190<br>102<br>64 | 1/10<br>3/10<br>0/10<br>0/10 |
| Contrôle positif : 5-Fluorouracile | 20 | +1,2 | 20 | 173 | 0/10 |
| Contrôle négatif | - | +1,0 | 11,56 | - | 0/20 |
| Dérivé N° 15 | 1,25<br>2,5<br>5<br>10 | -1,9<br>-1,9<br>-2,5<br>-4,1 | 18,0<br>20,25<br>26<br>9,3 | 164<br>184<br>236<br>85 | 0/10<br>0/10<br>1/10<br>0/10 |
| Contrôle positif 5-Fluorouracile | 20 | -0,2 | 17 | 154 | 0/10 |
| Contrôle négatif | - | -0,95 | 11,0 | | 0/20 |
| Dérivé N° 17 | 24 | 0,0 | 15,4 | 131 | 0/10 |
| Contrôle négatif | - | -0,1 | 11,8 | - | 0/25 |
| Dérivé N°19 | 5<br>10<br>20<br>40 | +0,5<br>-0,2<br>+0,3<br>-2,1 | 15,8<br>17,0<br>20,3<br>15,3 | 142<br>153<br>182<br>137 | 0/6 *<br>0/6 *<br>0/6 *<br>0/6 * |
| Contrôle positif : 5-Fluorouracile | | | | | |
| Contrôle négatif | - | +0,1 | 11,1 | - | 0/23 |

* Exceptionnellement cette expérience a été réalisée sur des lots de 6 animaux traités.

b) Test de la leucémie P388, IP/IP, J1 (GERAN et al Cancer chemother., 1972, *2*, 07—57).

Le test est réalisé selon le protocole précédent, le composé à tester étant uniquement administré au jour J1.

Les résultats obtenus sont consignés dans le tableau no 2 ci-après.

TABLEAU N° 2 :

| Produit testé | Dose (mg/kg) | Variation pondérale (g) | Jour médian de survie | T/C % | Nombre de survivants |
|---|---|---|---|---|---|
| Dérivé N° 7 | 10 | +1,2 | 15,1 | 129 | 0/10 |
| | 20 | +0,2 | 16,25 | 139 | 0/10 |
| | 40 | -0,2 | 15,9 | 136 | 0/10 |
| | 80 | -1,7 | 2,3 | 20 | 0/10 |
| Contrôle négatif | - | +1,1 | 11,7 | - | 0/20 |
| Dérivé N° 10 | 5 | -0,1 | 15,3 | 139 | 0/10 |
| | 10 | +0,3 | 15,25 | 139 | 0/10 |
| | 20 | -0,8 | 15,6 | 142 | 0/10 |
| | 40 | -1,4 | 15,3 | 139 | 0/10 |
| Contrôle positif : 5-Fluorouracile | 20 | -3,2 | 19,0 | 173 | 0/10 |
| Contrôle négatif | - | +0,8 | 11,0 | - | 0/20 |

(Suite Tab. N° 2)

| Produit testé | Dose (mg/kg) | Variation pondérale (g) | Jour médian de survie | T/C % | Nombre de survivants |
|---|---|---|---|---|---|
| Dérivé N° 12 | 5 | +0,1 | 15,87 | 139 | 0/10 |
| | 10 | -0,6 | 17,75 | 156 | 0/10 |
| | 20 | -1,2 | 20,75 | 182 | 0/10 |
| | 40 | -5,2 | 23,0 | 202 | 1/10 |
| Dérivé N° 14 | 5 | -1,2 | 19,75 | 173 | 0/10 |
| | 10 | -1,4 | 19,0 | 167 | 1/10 |
| | 20 | -2,5 | 18,75 | 164 | 1/10 |
| Contrôle négatif | - | -0,05 | 11,4 | - | 0/20 |

c) Test de la leucémie L 1210, IP/IP, J1—5 (GERAN et al, Cancer Chemother., 1972, *2*, 07—57).

Les essais sont effectués avec des souris hybrides $CDF_1$ inoculées par voie intrapéritonéale avec $10^5$ cellules leucémiques de la lignée L 1210. Le composé à tester est administré pendant 5 jours ($J_1$—$J_5$) par voie intrapéritonéale. Le contrôle positif du test est 5-Fluorouracile administré dans les mêmes conditions. Le nombre d'animaux survivants est évalué au jour $J_{60}$. Un composé est considéré comme étant actif lorsque le rapport T/C est supérieur ou égale à 125%.

Les résultats obtenus figurent dans le tableau no 3 suivant:

TABLEAU N°3 :

| Proauit testé | Dose (mg/kg) | Variation pondérale (g) | Jour médian de survie | T/C % | Nombre de survivants |
|---|---|---|---|---|---|
| Dérivé N°7 | 0,625 | -0,9 | 11,75 | 133 | 0/10 |
| | 1,25 | -1,2 | 14,25 | 162 | 0/10 |
| | 2,5 | -1,1 | 20,75 | 236 | 4/10 |
| | 5,0 | -0,8 | 14,0 | 159 | 2/10 |
| | 10 | -2,2 | 37,0 | 420 | 5/10 |
| | 20 | -4,2 | 35,0 | 398 | 5/10 |
| | 30 | -5,6 | 8,25 | 94 | 0/10 |
| Contrôle positif : 5-Fluorouracile | 20 | -0,5 | 16,0 | 182 | 0/10 |
| Contrôle négatif : | - | -0,8 | 8,8 | - | 0/25 |
| Dérivé N°10 | 1,25 | -1,7 | 12,75 | 145 | 1/10 |
| | 2,5 | -2,1 | 20,0 | 227 | 4/10 |
| | 5 | -2,7 | >60 | >682 | 6/10 |
| | 10 | -3,1 | 15,25 | 173 | 4/10 |
| | 20 | -5,6 | 8,4 | 95 | 0/10 |
| Contrôle positif : 5-Fluorouracile | 20 | -0,5 | 16,0 | 182 | 0/10 |
| Contrôle négatif : | - | -0,8 | 8,8 | - | 0/25 |

d) Test de mélanome B 16 (GERAN et al, Cancer Chemother., 1972, *2*, 07—57). Les essais sont effectués avec des souris hybrides $BDF_1$ inoculées par voie intrapéritonéale avec 0,5 ml d'un homogénat de mélanome B 16 réalisé à partir de 1 g de tumeur dans 10 ml de sérum physiologique. Le composé à tester est administré pendant 9 jours ($J_1$—$J_9$) par voie intrapéritoneale. Le contrôle positif du test est le Cis-Platine

administré dans les mêmes conditions. Le nombre d'animaux survivants est évalué au jour $J_{60}$. Un composé est considéré comme étant actif lorsque le rapport T/C est supérieur ou égale à 125%.

Les résultats obtenus sont rassemblés dans le tableau no 4 suivant:

TABLEAU N° 4

| Produit testé | Dose (mg/kg) | Variation pondérale (g) | Jour médian de survie | T/C % | Nombre de survivants |
|---|---|---|---|---|---|
| Dérivé N° 4 | 5 | -1,9 | 29,0 | 135 | 0/10 |
| Contrôle négatif | - | -1,0 | 21,5 | - | 0/20 |
| Dérivé N° 7 | 2,5 | +3-6 | 34 | 124 | 0/10 |
| | 5 | -0,1 | 48 | 174 | 0/10 |
| | 10 | +1,6 | 56,25 | 205 | 4/10 |
| | 20 | -0,8 | 58 | 211 | 5/10 |
| | 30 | -1,0 | 9,0 | 33 | 0/10 |
| Contrôle positif : Cis-Platine | 0,5 | -0,6 | 36 | 131 | 0/10 |
| Contrôle négatif | - | +2,1 | 27,5 | - | 0/25 |
| Dérivé N° 9 | 0,625 | +1,3 | 28 | 118 | 0/10 |
| | 1,25 | +0,1 | 33,0 | 139 | 1/10 |
| | 2,5 | +0,8 | 31,25 | 131 | 1/10 |
| Contrôle positif Cis-Platine | 0,5 | -0,6 | 30,0 | 126 | 0/10 |
| Contrôle négatif | - | +0,8 | 23,8 | - | 0/30 |

(Suite Tab. N° 4)

| Produit testé | Dose (mg/kg) | Variation pondérale (g) | Jour médian de survie | T/C % | Nombre de survivants |
|---|---|---|---|---|---|
| Dérivé N° 10 | 1,25<br>2,5<br>5<br>10 | +2,8<br>+1,8<br>+2,4<br>+0,5 | 32,0<br>38,75<br>44,0<br>11,7 | 116<br>141<br>160<br>42 | 0/10<br>0/10<br>0/10<br>0/10 |
| Contrôle positif : Cis-Platine | 0,5 | -0,6 | 38,75 | 141 | 0/10 |
| Contrôle négatif | - | +2,1 | 27,5 | - | 0 |
| Dérivé N° 11 | 1,25<br>2,5<br>5<br>10 | -2,5<br>+0,6<br>+0,2<br>-1,0 | 32,25<br>35,25<br>41,25<br>19,3 | 133<br>145<br>170<br>79 | 0/10<br>0/10<br>3/10<br>0/10 |
| Dérivé N° 14 | 1,25<br>2,5<br>5<br>10 | +1,0<br>+0,1<br>-0,2<br>-1,9 | 29,75<br>32,6<br>50,0<br>11,75 | 123<br>134<br>206<br>48 | 0/10<br>0/10<br>1/10<br>0/10 |
| Contrôle négatif | - | -2,0 | 24,75 | - | 0/25 |

e) Test de réticulosarcome M5-M5076 (N.C.I. Protocol 3 M., 531, p 1—7, 23.5.1983).

Les essais sont effectués avec des souris hybrides $BDF_1$ inoculées par voie intrapéritonéale avec $10^6$ cellules de la lignée M5-M5076. Le composé à tester est administré 5 fois successivement de façon séquentielle (J1, 5, 9, 13, 17) par voie intrapéritoneale. Le contrôle positif du test est le cyclophosphamide administré dans les mêmes conditions. Le nombre d'animaux survivants est évalué au jour $J_{75}$. Un composé est considéré comme actif lorsque le rapport T/C est supérieur ou égale à 125%.

Les résultats obtenus sont consignés dans le tableau no 5 suivant:

TABLEAU N° 5

| Produit testé | Dose (mg/kg) | Variation pondérale (g) | Jour médian de survie | T/C % | Nombre de survivants |
|---|---|---|---|---|---|
| Dérivé N° 1 | 50 | -0,6 | 32,7 | 130 | 0/10 |
| Contrôle positif : Cyclophospnamiae | 160 | -1,9 | > 60 | > 239 | 9/10 |
| Contrôle négatif | - | +1,8 | 25,1 | - | 0/40 |
| Dérivé N° 7 | 2,5 | -1,0 | 31,25 | 120 | 0/10 |
| | 5 | -1,4 | 33,0 | 127 | 1/10 |
| | 10 | -1,8 | 37,25 | 143 | 0/10 |
| | 20 | -1,9 | 41,0 | 158 | 0/10 |
| | 40 | -1,8 | 42,0 | 161 | 1/10 |
| Contrôle positif : Cyclophosphamide | 160 | -4,9 | 57,0 | 219 | 0/10 |
| Contrôle négatif | - | -0,8 | 26,0 | - | 0/30 |
| Dérivé N° 10 | 1,25 | +2,7 | 34,0 | 133 | 1/10 |
| | 2,5 | +0,4 | 33,0 | 129 | 1/10 |
| | 5 | +2,1 | 40,0 | 156 | 1/10 |
| | 10 | +1,2 | 42,0 | 164 | 1/10 |
| | 20 | 0,0 | 44,75 | 175 | 1/10 |
| | 40 | -2,8 | 46,0 | 180 | 2/10 |
| Contrôle positif : Cyclophospnamide | 160 | -1,9 | > 60 | > 234 | 9/10 |
| Contrôle négatif | - | +1,0 | 25,6 | - | 0/20 |

f) Test de la leucémie P 388 IP/J1 IV (GERAN et al, Cancer Chemother., 1972, *2*, 07—57.

Le test est réalisé selon le même protocole que celui décrit pour le test a), le composé à tester étant administré par voie intraveineuse uniquement au jour $J_1$.

Les résultats obtenus sont consignés dans le tableau no 6 ci-après.

TABLEAU N° 6 :

| Produit testé | Dose mg/kg | Variation pondérale (g) | Jour médian de survie | T/C % | Nombre de survivants |
|---|---|---|---|---|---|
| Dérivé n° 12 | 50<br>100 | - 0,7<br>- 1,6 | 19,0<br>24,3 | 174<br>223 | 0/10<br>1/10 |
| Dérivé n° 15 | 50<br>100 | + 0,5<br>- 0,25 | 17,75<br>25,75 | 163<br>236 | 1/10<br>4/10 |
| Contrôle négatif | - | + 3,5 | 10,90 | - | - |

g) Test de la leucémie L 1210 IP/J1 IV (GERAN et al, Cancer Chemother., 1972, *2*, 07—57.

Le test est réalisé selon le même protocole que celui décrit pour le test c), le composé à tester étant administré par voie intraveineuse uniquement au jour $J_1$.

Les résultats obtenus sont consignés dans le tableau no 7 suivant:

TABLEAU N° 7 :

| Produit testé | Dose mg/kg | Variation pondérale (g) | Jour médian de survie | T/C % | Nombre de survivants |
|---|---|---|---|---|---|
| Dérivé n° 11 | 20<br>40 | - 0,4<br>- 0,2 | 11,8<br>13,3 | 132<br>149 | 0/10<br>0/10 |
| Dérivé n° 14 | 20 | - 1,1 | 14,0 | 157 | 0/10 |
| Contrôle négatif | - | + 0,8 | 8,9 | - | 0/15 |

h) Test de l'adénocarcinome colique 38, SC/J1 IV (GERAN et al, Cancer Chemother., 1972, *2*, 07—57.

Les essais sont effectués avec de souris hybrides $BDF_1$ inoculée par voie sous cutanée avec un fragment tumoral standardisé de 3 $mm^3$.

Le composé à tester est administré par voie intraveineuse uniquement au jour $J_1$.
Le nombre des animaux survivants est évalué au jour $J_{38}$.
Les résultats obtenus sont rassemblés dans le tableau no 8.

TABLEAU N° 8 :

| Produit testé | Dose mg/kg | Volume tumoral moyen à J 38 | T/C % à J38 | Nombre de survivants |
|---|---|---|---|---|
| Dérivé n° 12 | 100 | 3,24 | 36 | 10/10 |
| Dérivé n° 15 | 50 | 2,73 | 30 | 10/10 |
| Contrôle négatif | - | 8,94 | - | 5/10 |

i) Test de l'adénocarcinome colique 38, SC/$J_{2,9}$ IV (GERAN et al, Cancer Chemotherap., 1972, *2*, 07—57.
Ce test est réalisé selon le protocole décrit pour le test h, le coposé à tester étant administré par voie intraveineuse au jour $J_2$ puis au jour $J_9$.
Le nombre des animaux survivants est évalué au jour $J_{32}$.
Les résultats obtenus sont consignés dans le tableau no 9 suivant:

TABLEAU N° 9 :

| Produit testé | Dose mg/kg | Volume tumoral moyen à J 32 | T/C % à J32 | Nombre de survivants |
|---|---|---|---|---|
| Dérivé n° 12 | 50<br>75 | 1,38<br>0,97 | 18<br>13 | 10/10<br>10/10 |
| Dérivé n° 15 | 25<br>50 | 0,0<br>0,0 | 0<br>0 | 10/10 |
| Contrôle négatif | - | 7,51 | - | 5/10 |

Les études toxicologique et pharmacologique qui viennent d'être rapportées ont mis en évidence les intéressantes propriétés antitumorales des composés de l'invention qui les rendent très utiles en thérapeutique.

Le médicament de l'invention peut-être présenté pour l'administration orale sous forme de comprimés, comprimés dragéifiés, gélules, gouttes ou sirop. Il peut aussi être présenté pour l'administration rectale sous forme de suppositoires et pour l'administration parentérale sous forme de soluté injectable par voie intramusculaire ou intraveineuse.

Chaque dose unitaire contient avantageusement de 0,005 g à 0,400 g associés à des excipients et véhicules convenables, les doses administrables journellement peuvent varier de 0,005 g à 3,00 g de principe actif, en fonction du poids du malade, de son âge et de la sévérité de son état.

On donnera, ci-après, à titre d'exemple non limitatif, quelques formulations pharmaceutiques du médicament de l'invention.

1) Comprimés dragéifiés
Dérivé no 4 0,250 g
Excipient: kaolin, lactose, carbonate de magnésium, gomme arabique, amidon de maïs, saccharose, géaltine, cire blanche.

2) Comprimés
Dérivé no 9 0,300 g
Excipient: amidon de maîs, lactose, stéarate de magnesium, talc.

3) Gélules
Dérivé no 1 0,150 g
Excipient: stéarate de magnésium, talc, aérosil.

4) Suppositories
Dérivé no 7 0,250 g
Excipient: triglcérides semi-synthétiques q.s.p. 1 suppositoire.

5) Soluté injectable
Dérivé no 11 (bimaléate) 0,400 g
Dérivé no 15 (bichlorhdyrate) 0,100 g
Excipient: solvant isotonique q.s.p. 10 ml

Pour ses propriétés antitumorales, le médicament de l'invention est indiqué dans le traitement des tumeurs solides et de leurs métastases ainsi que dans le traitement des leucémies.

**Revendications pour les Etats Contractants: BE CH DE FR GB IT LI LU NL SE**

1. Composés de formule (I)

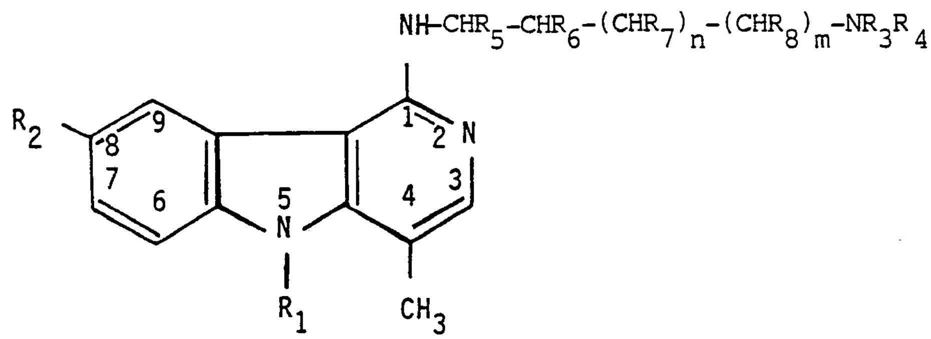

(I)

dans laquelle n et m sont indépendamment 0 ou 1, $R_1$ représente l'hydrogène, ou un groupe alkyle en $C_1$—$C_4$, $R_2$ représente l'hydrogène, un groupe hydroxy ou un groupe alkoxy en $C_1$—$C_4$, $R_3$ et $R_4$ sont, chacun indépendamment l'un de l'autre l'hydrogène, un groupe alkyle ou hydroxyalkyle en $C_1$—$C_4$; $R_5$, $R_6$, $R_7$ et $R_8$ représentent indépendamment l'hydrogène, un groupe hydroxy ou un groupe alkyle en $C_1$—$C_4$, ainsi que les formes tautomères lorsqu'elles existent et les sels d'addition avec les acides minéraux ou organiques pharmaceutiquement acceptables.

2. (Diéthylamino-3 propylamino)-1 méthyl-4 5-H pyrido [4,3-b] indole et ses sels pharmaceutiquement acceptables.

3. (Diéthylamino-3 propylamino)-1 méthoxy-8 méthyl-4 5-H pyrido [4,3-b] indole et ses sels pharmaceutiquement acceptables.

4. (Diéthylamino-3 propylamino)-1 hydroxy-8 méthyl-4 5-H pyrido [4,3-b] indole et ses sels pharmaceutiquement acceptables.

5. (Diméthylamino-3 propylamino)-1 hydroxy-8 méthyl-4 5-H pyrido [4,3-b] indole et ses sels pharmaceutiquement acceptables.

6. (Diéthylamino-3 propylamino)-1 diméthyl-4,5 hydroxy-8 5-H pyrido [4,3-b] indole et ses sels pharmaceutiquement acceptables.

7. Diméthyl-4,5 (diméthylamino-3 propylamino)-1 hydroxy-8 5-H pyrido [4,3-b] indole et ses sels pharmaceutiquement acceptables.

8. Diméthyl-4,5 (diméthylamino-3 méthyl-2 propylamino)-1 hydroxy-8 5-H pyrido [4,3-b] indole et ses sels pharmaceutiquement acceptables.

9. (Diéthylamino-2 éthylamino)-1 diméthyl-4,5 hydroxy-8 5-H pyrido [4,3-b] indole et ses sels pharmaceutiquement acceptables.

10. Procédé de préparation des composés selon la revendication 1, caractérisé en ce que: la méthyl-4 2-H 5-H pyrido [4,3-b] indolone-1 de formule (IV):

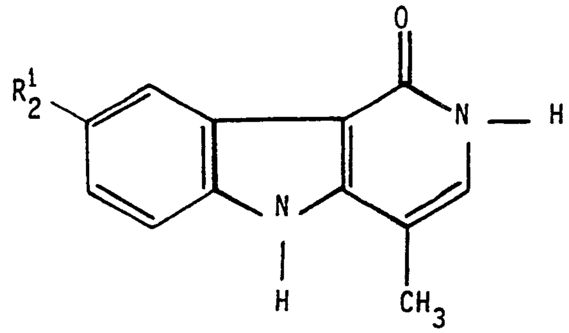

(IV)

dans laquelle R'$_2$ représente l'hydrogène ou un radical alkoxy en $C_1$—$C_4$, est transformée par action d'un agent chlorant en un chloro-1 méthyl-4 5-H pyrido [4,3-b] indole de formule (V)

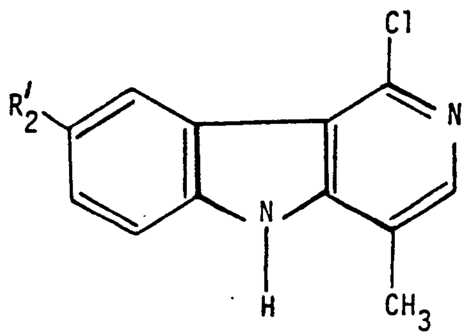

(V)

dans laquelle R'$_2$ représente l'hydrogène ou un radical alkoxy en $C_1$—$C_4$, puis le composé précédent éventuellement après alkylation en position 5, au moyen d'un halogénure d'alkyle en $C_1$—$C_4$ et éventuellement après transformation en composé hydroxylé correspondant (V, R'$_2$ = OH) par action d'un acide minéral, est condensé avec une amine de formule:

$$H_2N—CHR_5—CHR_6—(CHR_7)_n—(CHR_8)_m—NR_3R_4$$

dans laquelle n, m, $R_3$, $R_4$, $R_5$, $R_6$, $R_7$ et $R_8$ sont tels que définis dans la revendication 1 pour le former le composé de formule (I).

11. Médicament caractérisé en ce qu'il contient à titre de principe actif un dérivé selon l'une quelconque des revendications 1 à 9.

12. Médicament selon la revendication 11, caractérisé en ce que chaque dose unitaire contient 0,005 g à 0,400 g de principe actif.

13. Méthyl-4 2-H 5-H pyrido (4,3-b] indolone-1 de formule IV

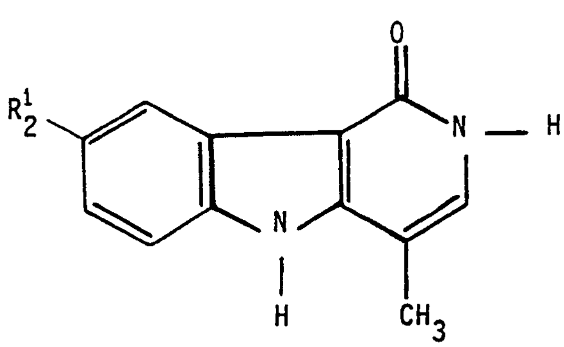

(IV)

dans laquelle $R'_2$ représente l'hydrogène ou un groupe alkoxy en $C_1$—$C_4$.

14. Procédé de préparation d'un composé de formule IV selon la revendication 13, caractérisé en ce que l'hydroxy-4 méthyl-5 1-H pyridone-2 de formule

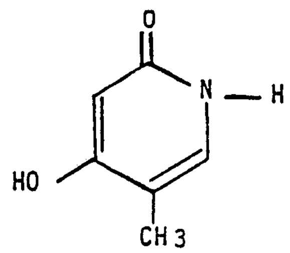

est mise à réagir à haute température avec une phénylhydrazine de formule

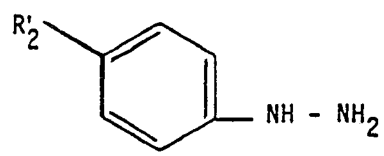

dans laquelle $R'_2$ a la même signification que dans la formule IV.

**Revendications pour les Etats Contractants: AT ES GR**

1. Procédé de préparation de composés de formule (I)

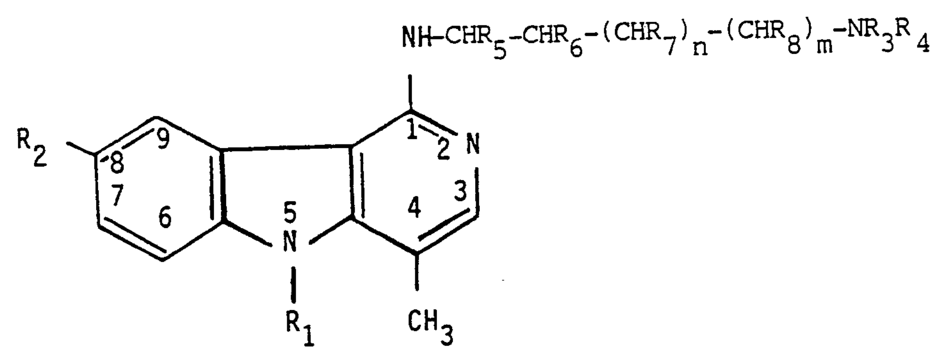

(I)

dans laquelle n et m sont indépendamment 0 ou 1, $R_1$ représente l'hydrogène, ou un groupe alkyle en $C_1$—$C_4$, $R_2$ représente l'hydrogène, un groupe hydroxy ou un groupe alkoxy en $C_1$—$C_4$, $R_3$ et $R_4$ sont, chacun indépendamment l'un de l'autre l'hydrogène, un groupe alkyle ou hydroxyalkyle en $C_1$—$C_4$; $R_5$, $R_6$, $R_7$ et $R_8$ représentent indépendamment l'hydrogène, un groupe hydroxy ou un groupe alkyle en $C_1$—$C_4$, ainsi que les formes tautomères lorsqu'elles existent et les sels d'addition avec les acides minéraux ou organiques pharmaceutiquement acceptables, caractérisé en ce que la méthyl-4 2-H 5-H pyrido [4,3-b] indolone-1 de formule (IV):

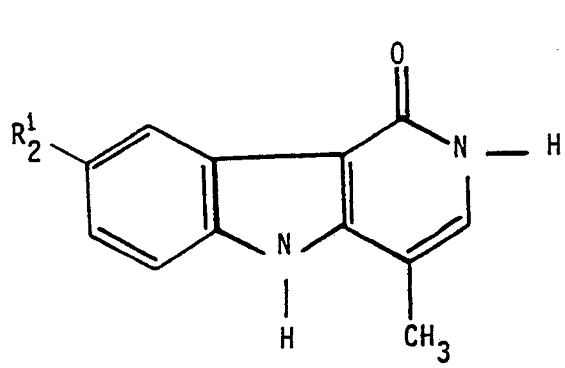

(IV)

dans laquelle $R'_2$ représente l'hydrogène ou un radical alkoxy en $C_1$—$C_4$, est transformée par action d'un agent chlorant en un chloro-1 méthyl-4 5-H pyrido [4,3-b] indole de formule (V)

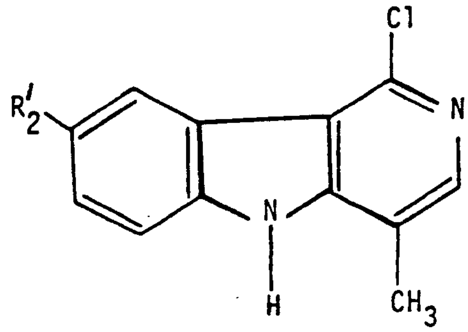

(V)

dans laquelle $R'_2$ représente l'hydrogène ou un radical alkoxy en $C_1$—$C_4$, puis le composé précédent éventuellement après alkylation en position 5, au moyen d'un halogénure d'alkyle en $C_1$—$C_4$ et éventuellement après transformation en composé hydroxylé correspondant (V, $R'_2$ = OH) par action d'un acide minéral, est condensé avec une amine de formule:

$$H_2N—CHR_5—CHR_6—(CHR_7)_n—(CHR_8)_m—NR_3R_4$$

dans laquelle n, m, $R_3$, $R_4$, $R_5$, $R_6$, $R_7$ et $R_8$ sont tels que définis dans la revendication 1 pour le former le composé de formule (I).

2. Procédé de préparation d'un médicament, caractérisé en ce qu'on met sous une forme pharmaceutiquement acceptable un composé obtenu selon la revendication 1.

3. Procédé de préparation d'une méthyl-4 2-H 5-H pyrido[4,3-b]indolone-1 de formule IV

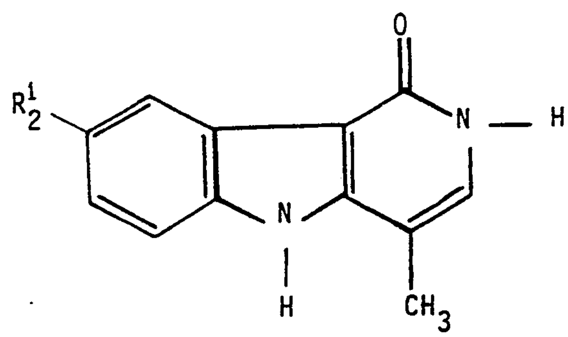

(IV)

dans laquelle $R'_2$ représente l'hydrogène ou un groupe alkoxy en $C_1$—$C_4$, caractérisé en ce que l'hydroxy-4 méthyl-5 1-H pyridone-2 de formule

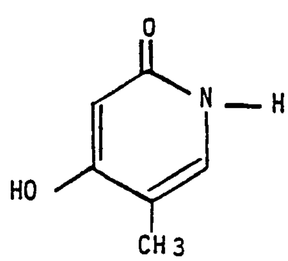

est mise à réagir à haute température avec une phénylhydrazine de formule

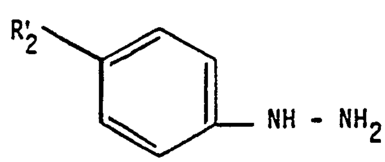

dans laquelle $R'_2$ a la même signification que dans la formule IV.

**Patentansprüche für die Vertragsstaaten: BE CH DE FR GB IT LI LU NL SE**

1. Verbindungen der Formel (I)

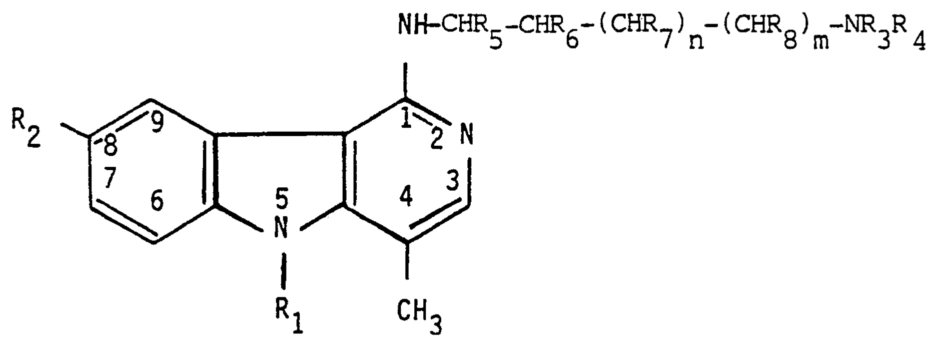

(I)

in der n und m unabhängig voneinander 0 oder 1, $R_1$ ein Wasserstoffatom oder eine $C_1$—$C_4$-Alkylgruppe, $R_2$ ein Wasserstoffatom, eine Hydroxygruppe oder eine $C_1$—$C_4$-Alkoxygruppe, $R_3$ und $R_4$ jeweils unabhängig voneinander Wasserstoffatome, $C_1$—$C_4$-Alkylgruppen oder $C_1$—$C_4$-Hydroxyalkylgruppen und $R_5$, $R_6$, $R_7$ und $R_8$ jeweils unabhängig voneinander Wasserstoffatome, Hydroxygruppen oder $C_1$—$C_4$-Alkylgruppen bedeuten, sowie deren tautomere Formen, soweit sie existieren und deren Additionssalze mit anorganischen oder organischen, pharmazeutisch annehmbaren Säuren.

2. 1-(3-Diethylamino-propylamino)-4-methyl-5H-pyrido[4,3-b]indol und dessen pharmazeutisch annehmbare Salze.

3. 1-(3-Diethylamino-propylamino)-8-methoxy-4-methyl-5H-pyrido[4,3-b]indol und dessen pharmazeutisch annehmbare Salze.

4. 1-(3-Diethylamino-propylamino)-8-hydroxy-4-methyl-5H-pyrido[4,3-b]indol und dessen pharmazeutisch annehmbare Salze.

5. 1-(3-Dimethylamino-propylamino)-8-hydroxy-4-methyl-5H-pyrido[4,3-b]indol und dessen pharmazeutisch annehmbare Salze.

6. 1-(3-Dimethylamino-propylamino)-4,5-dimethyl-8-hydroxy-5H-pyrido[4,3-b]indol und dessen pharmazeutisch annehmbare Salze.

7. 1-(3-Dimethylamino-propylamino)-4,5-dimethyl-8-hydroxy-5H-pyrido[4,3-b]indol und dessen pharmazeutisch annehmbare Salze.

8. 1-(3-Dimethylamino-2-methyl-propylamino)-4,5-dimethyl-8-hydroxy-5H-pyrido[4,3-b]indol und dessen pharmazeutisch annehmbare Salze.

9. 1-(2-Diethylamino-ethylamino)-4,5-dimethyl-8-hydroxy-5H-pyrido[4,3-b]indol und dessen pharmazeutisch annehmbare Salze.

10. Verfahren zur Herstellung der Verbindung nach Anspruch 1, dadurch gekennzeichnet, daß man das 4-Methyl-2H-5H-pyrido[4,3-b]indolon-1 der Formel (IV)

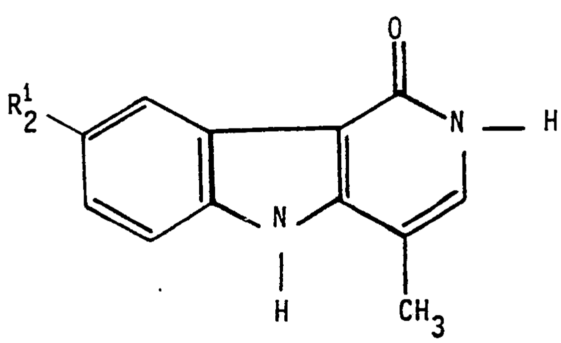

(IV)

in der $R'_2$ ein Wasserstoffatom oder eine $C_1$—$C_4$-Alkoxygruppe bedeutet, durch Einwirkung eines Chlorierungsmittels in ein 1-Chlor-4-methyl-5H-pyrido[4,3-b]indol der Formel (V)

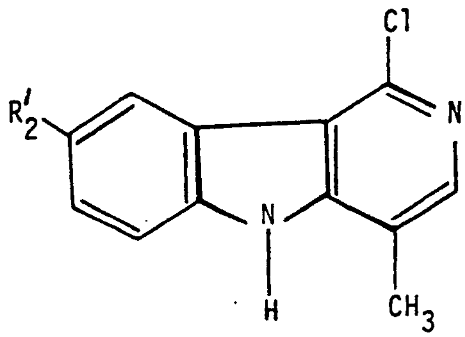

(V)

in der $R'_2$ ein Wasserstoffatom oder eine $C_1$—$C_4$-Alkoxygruppe bedeutet, umwandelt, und dann die erhaltene Verbindung gegebenenfalls nach der Alkylierung in der 5-Stellung mit einem $C_1$—$C_4$-Alkylhalogenid alkyliert und gegebenenfalls nach der Umwandlung durch Einwirkung einer anorganischen Säure in die entsprechende Hydroxyverbindung (V, $R'_2$ = OH) mit einen Amin der Formel

$$H_2N—CHR_5—CHR_6—(CHR_7)_n—(CHR_8)_m—NR_3R_4$$

in der n, m, $R_3$, $R_4$, $R_5$, $R_6$, $R_7$ und $R_8$ die in Anspruch 1 angegebenen Bedeutungen besitzen, kondensiert zur Bildung der Verbindung der Formel (I).

11. Arzneimittel, dadurch gekennzeichnet, daß es als Wirkstoff ein Derivat nach einem der Ansprüche 1 bis 9 enthält.

12. Arzneimittel nach Anspruch 11, dadurch gekennzeichnet, daß jede Einheitsdosis 0,005 bis 0,400 g des Wirkstoffes enthält.

13. 4-Methyl-2H-5H-pyrido[4,3-b]indolon-1 der Formel (IV)

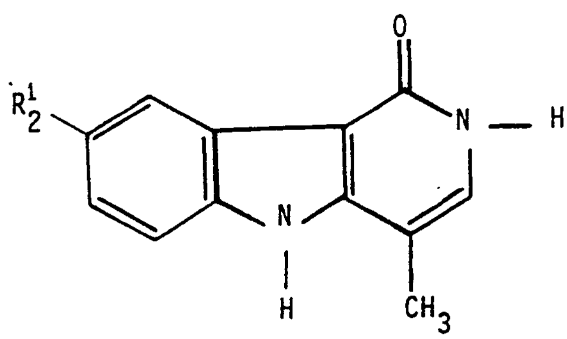

(IV)

in der $R'_2$ ein Wasserstoffatom oder eine $C_1$—$C_4$-Alkoxygruppe bedeutet.

14. Verfharen zur Herstellung einer Verbindung der Formel (IV) nach Anspruch 13, dadurch gekennzeichnet, daß man 4-Hydroxy-5-methyl-1H-pyridon-2 der Formel

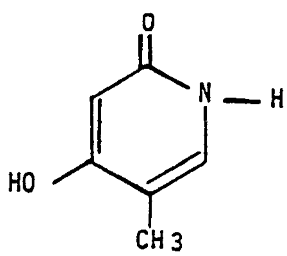

bei hoher Temperatur mit einem Phenylhydrazin der Formel

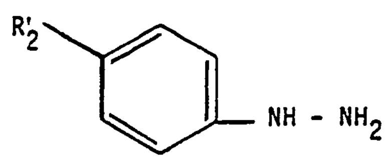

in der $R'_2$ die bezüglich der Formel IV angegebenen Bedeutungen besitzt, umsetzt.

**Patentansprüche für die Vertragsstaaten: AT ES GR**

1. Verfahren zur Herstellung von Verbindungen der Formel (I)

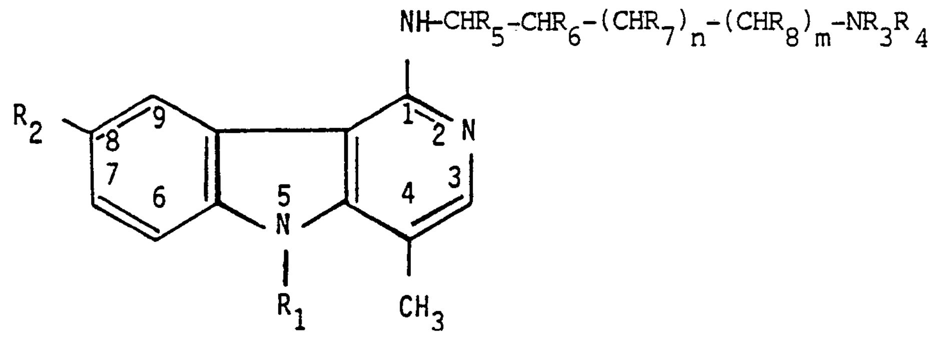

(I)

in der n und m unabhängig voneinander 0 oder 1, $R_1$ ein Wasserstoffatom oder eine $C_1$—$C_4$-Alkylgruppe, $R_2$ ein Wasserstoffatom, eine Hydroxygruppe oder eine $C_1$—$C_4$-Alkoxygruppe, $R_3$ und $R_4$ jeweils unabhängig voneinander Wasserstoffatome, $C_1$—$C_4$-Alkylgruppen oder $C_1$—$C_4$-Hydroxyxalkylgruppen und $R_5$, $R_6$, $R_7$ und $R_8$ jeweils unabhängig voneinander Wasserstoffatome, Hydroxygruppen oder $C_1$—$C_4$-Alkylgruppen bedeuten, sowie deren tautomeren Formen, soweit sie existieren und deren Additionssalze mit anorganischen oder organischen, pharmazeutisch annehmbaren Säuren, dadurch gekennzeichnet, daß daß man das 4-Methyl-2H-5H-pyrido[4,3-b]indolon-1 der Formel (IV)

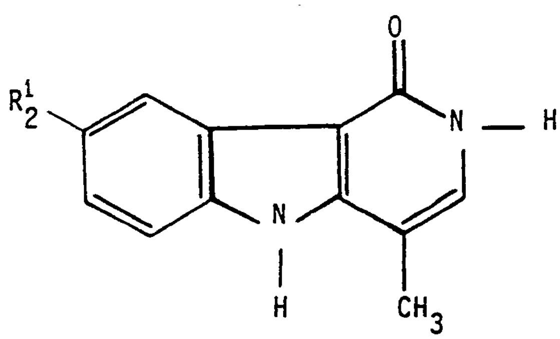

(IV)

in der $R'_2$ ein Wasserstoffatom oder eine $C_1$—$C_4$-Alkoxygruppe bedeutet, durch Einwirkung eines Chlorierungsmittels in ein 1-Chlor-4-methyl-5H-pyrido[4,3-b]indol der Formel (V)

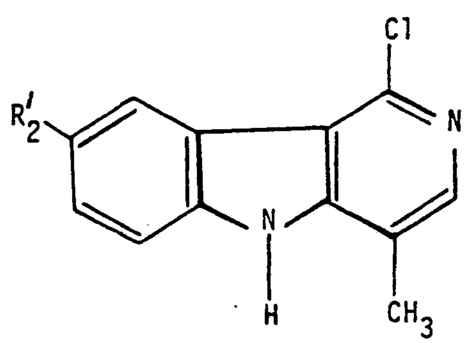

(V)

in der $R'_2$ ein Wasserstoffatom oder eine $C_1$—$C_4$-Alkoxygruppe bedeutet, umwandelt, und dann die erhaltene Verbindung gegebenenfalls nach der Alkylierung in der 5-Stellung mit einem $C_1$—$C_4$-Alkylhalogenid alkyliert und gegebenenfalls nach der Umwandlung durch Einwirkung einer anorganischen Säure in die entsprechende Hydroxyverbindung (V, $R'_2$ = OH) mit einen Amin der Formel

$$H_2N—CHR_5—CHR_6—(CHR_7)_n—(CHR_8)_m—NR_3R_4$$

in der n, m, $R_3$, $R_4$, $R_5$, $R_6$, $R_7$ und $R_8$ die in Anspruch 1 angegebenen Bedeutungen besitzen, kondensiert zur Bildung der Verbindung der Formel (I).

2. Verfahren zur Herstellung eines Arzneimittels, dadurch gekennzeichnet, daß man eine nach Anspruch 1 erhaltene Verbindung in eine pharmazeutisch annehmbare Form bringt.

3. Verfahren zur Herstellung eines 4-Methyl-2H-5H-pyrido[4,3-b]indolone-1 der Formel (IV)

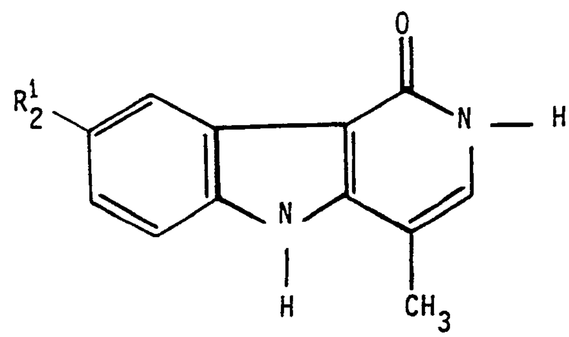

(IV)

in der $R'_2$ ein Wasserstoffatom oder eine $C_1$—$C_4$-Alkoxygruppe bedeutet, dadurch gekennzeichnet, daß man daß man 4-Hydroxy-5-methyl-1H-pyridon-2 der Formel

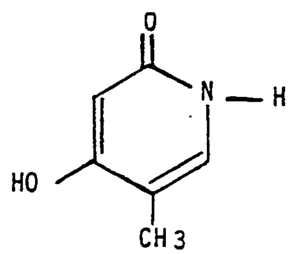

bei hoher Temperatur mit einem Phenylhydrazin der Formel

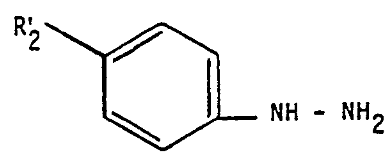

in der $R'_2$ die bezüglich der Formel IV angegebenen Bedeutungen besitzt, umsetzt.

**Claims for the Contracting States: BE CH DE FR GB IT LI LU NL SE**

1. Compounds of formula (I)

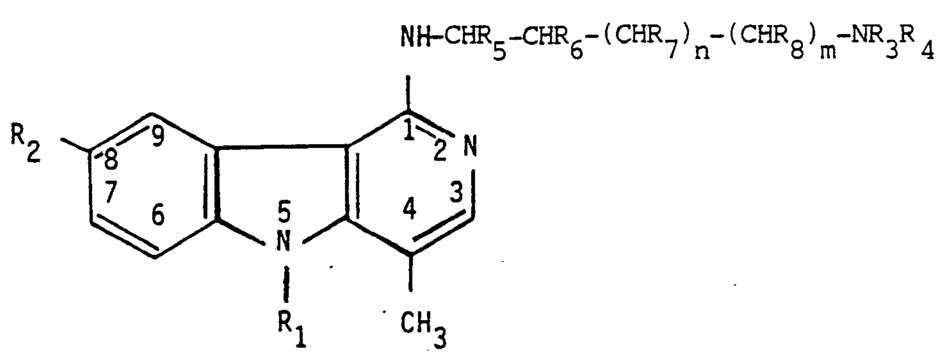

(I)

wherein n and m independently denote 0 or 1, $R_1$ represents hydrogen or $C_1$—$C_4$ alkyl, $R_2$ represents hydrogen, hydroxy or $C_1$—$C_4$ alkoxy, $R_3$ and $R_4$, independently of each other, denote hydrogen, $C_1$—$C_4$ alkyl or hydroxyalkyl, $R_5$, $R_6$, $R_7$ and $R_8$ independently denote hydrogen, hydroxy or $C_1$—$C_4$ alkyl, as well as the tautomeric forms when they exist, and the addition salts with pharmaceutically acceptable organic or inorganic acids.

2. 1-(3-Diethylamino-propylamino)-4-methyl-5H-pyrido[4,3-b]indole and the pharmaceutically acceptable salts thereof.

3. 1-(3-Diethylamino-propylamino)-8-methoxy-4-methyl-5H-pyrido[4,3-b]indole and the pharmaceutically acceptable salts thereof.

4. 1-(3-Diethylamino-propylamino)-8-hydroxy-4-methyl-5H-pyrido[4,3-b]indol and the pharmaceutically acceptable salts thereof.

5. 1-(3-Dimethylamino-propylamino)-8-hydroxy-4-methyl-5H-pyrido[4,3-b]indole and the pharmaceutically acceptable salts thereof.

6. 1-(3-Diethylamino-propylamino)-4,5-dimethyl-8-hydroxy-5H-pyrido[4,3-b]indole and the pharmaceutically acceptable salts thereof.

7. 4,5-Dimethyl-1-(3-dimethylamino-propylamino)-8-hydroxy-5H-pyrido[4,3-b]indole and the pharmaceutically acceptable salts thereof.

8. 4,5-Dimethyl-1(3-dimethylamino-2-methyl propylamino)-8-hydroxy-5H-pyrido[4,3-b]indole and the pharmaceutically acceptable salts thereof.

9. 1-(2-diethylamino-ethylamino)-4,5-dimethyl-8-hydroxy-5H-pyrido[4,3-b]indole and the pharmaceutically acceptable salts thereof.

10. Process for the preparation of compounds according to claim 1, characterized in that 4-methyl-2H,5H-pyrido[4,3]-1-indolone of the formula:

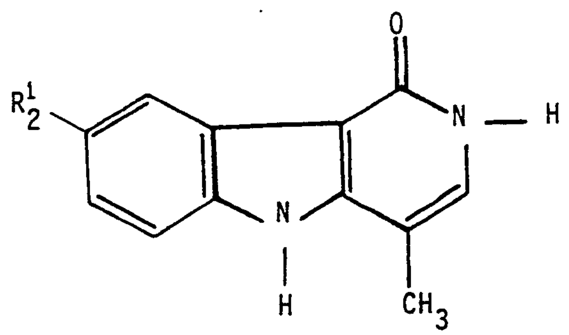

(IV)

in which $R'_2$ denotes hydrogen or $C_1$—$C_4$ alkoxy is converted by the action of a chlorinating agent into a 1-chloro-4-methyl-5H-pyrido[4,3-b]indole of formula (V):

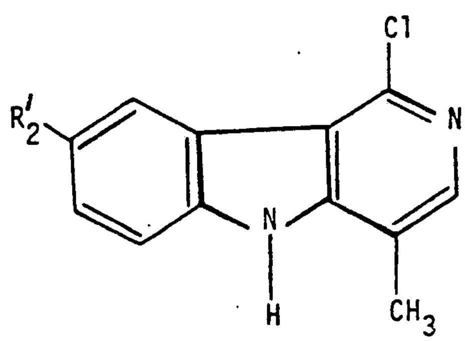

(V)

in which $R'_2$ denotes hydrogen or $C_1$—$C_4$ alkoxy, then the previous compound, possibly after alkylation in position 5 by means of a $C_{1-4}$ alkyl halide and possibly after conversion into a corresponding hydroxyl compound (V, $R'_2$ = OH) by the action of an inorganic acid, is condensed with an amine of the formula:

$$H_2N—CHR_5—CHR_6—(CHR_7)_n—(CHR_8)_m—NR_3R_4$$

in which n, m, $R_3$, $R_4$, $R_5$, $R_6$, $R_7$ and $R_8$ are as defined in claim 1, to form the compound of formula (I).

11. A pharmaceutical composition, characterized in that it contains as active principle as deriative according to any of claims 1 to 9.

12. A pharmaceutical composition according to claim 11, characterized in that each dosage unit contains 0.005 g to 0.400 g of active principle.

13. 4-methyl-2H-5H-pyrido[4,3-b]-1-indolone of formula IV

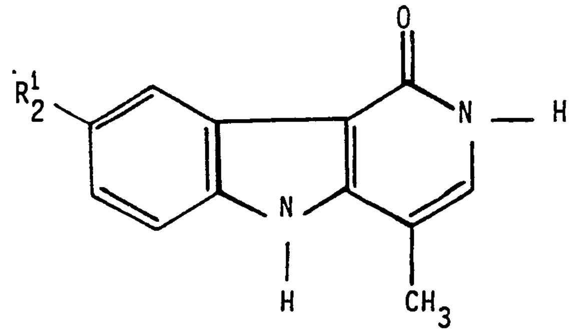

(IV)

wherein alkoxy presents hydrogen or a $C_{1-4}$ alkoxy group.

14. A process for preparing a compound of formula IV according to claim 13, characterised in that 4-hydroxy-5-methyl-1H-2-pyridone of formula

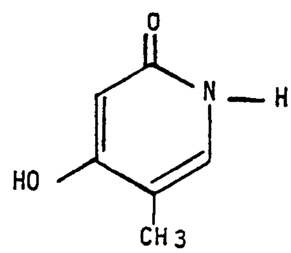

is reacted at high temperature with a phenylhydrazine of formula

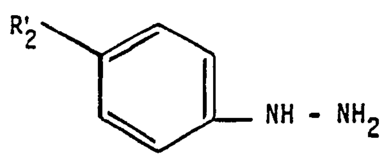

wherein $R'_2$ has the same meaning as in formula IV.

## Claims for the Contracting States: AT ES GR

1. Process for preparing compounds of formula (I)

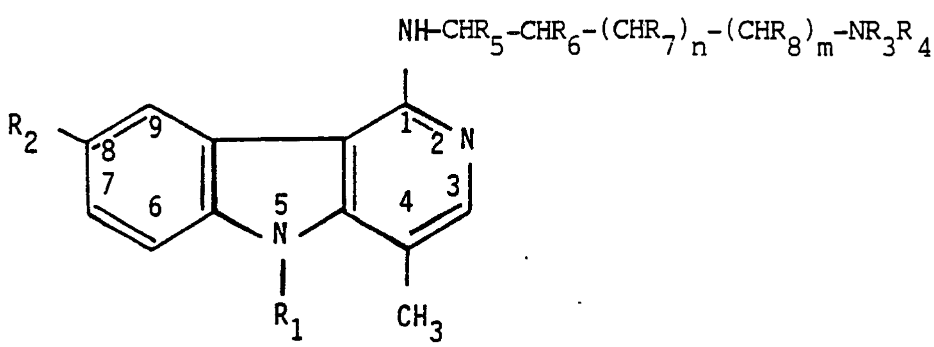

(I)

wherein n and m independently denote 0 or 1, $R_1$ represents hydrogen or $C_1$—$C_4$ alkyl, $R_2$ represents hydrogen, hydroxy or $C_1$—$C_4$ alkoxy, $R_3$ and $R_4$, independently of each other, denote hydrogen, $C_1$—$C_4$ alkyl or hydroxyalkyl, $R_5$, $R_6$, $R_7$ and $R_8$ independently denote hydrogen, hydroxy or $C_1$—$C_4$ alkyl, as well as the

tautomeric forms when they exist, and the addition salts with pharmaceutically acceptable organic or inorganic acids, characterized in that 4-methyl-2H,5H-pyrido[4,3]-1-indolone of the formula:

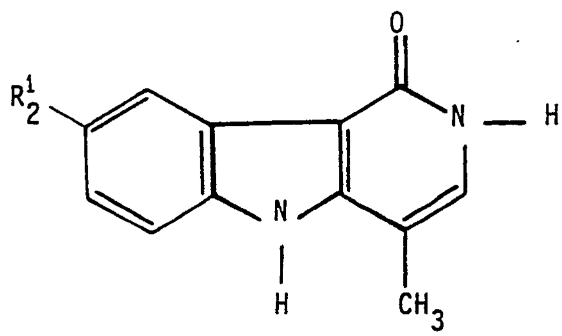

(IV)

in which R'$_2$ denotes hydrogen or $C_1$—$C_4$ alkoxy is converted by the action of a chlorinating agent into a 1-chloro-4-methyl-5H-pyrido[4,3-b]indole of formula (V):

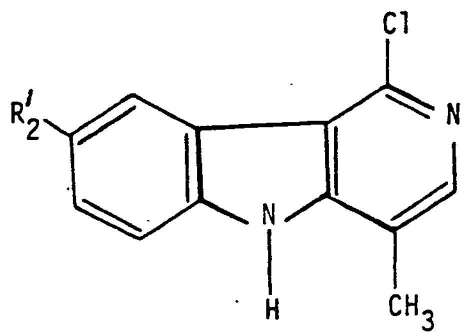

(V)

in which R'$_2$ denotes hydrogen or $C_1$—$C_4$ alkoxy, then the previous compound, possibly after alkylation in position 5 by means of a $C_{1-4}$ alkyl halide and possibly after conversion into a corresponding hydroxyl compound (V, R'$_2$ = OH) by the action of an inorganic acid, is condensed with an amine of the formula:

$$H_2N—CHR_5—CHR_6—(CHR_7)_n—(CHR_8)_m—NR_3R_4$$

in which n, m, $R_3$, $R_4$, $R_5$, $R_6$, $R_7$ and $R_8$ are as defined in claim 1, to form the compound of formula (I).

2. Process for preparing a pharmaceutical composition, characterized in that a compound obtained according to claim 1 is made into a pharmaceutically acceptable form.

3. Process for preparing a 4-methyl-2H-5H-pyrido[4,3-b]-1-indolone of formula IV

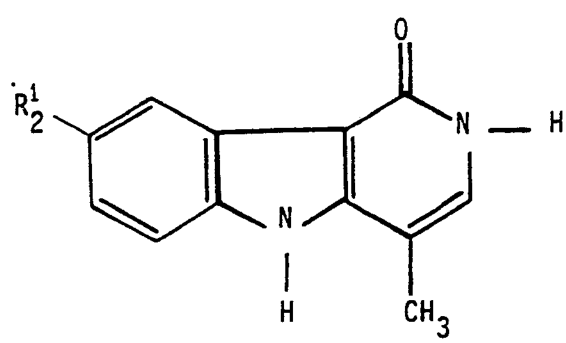

(IV)

wherein $R'_2$ represents hydrogen or a $C_{1-4}$ alkoxy group, characterised in that 4-hydroxy-5-methyl-1H-2-pyridone of formula

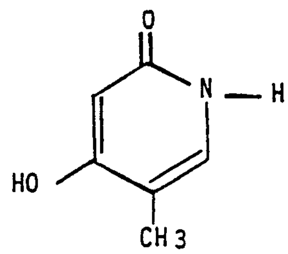

is reacted at high temperature with a phenylhydrazine of formula

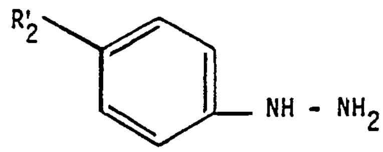

wherein $R'_2$ has the same meaning as in formula IV.